(19) Europäisches Patentamt
European Patent Office
Office européen des brevets

(11) **EP 4 366 638 B1**

(12) **EUROPEAN PATENT SPECIFICATION**

(45) Date of publication and mention
of the grant of the patent:
**10.12.2025 Bulletin 2025/50**

(21) Application number: **23738897.0**

(22) Date of filing: **27.06.2023**

(51) International Patent Classification (IPC):
**A61B 18/14** (2006.01)     **A61B 90/00** (2016.01)

(52) Cooperative Patent Classification (CPC):
**A61B 18/1445;** A61B 2018/00702;
A61B 2018/00827; A61B 2018/00875;
A61B 2018/00892; A61B 2018/1455;
A61B 2018/1467; A61B 2090/065

(86) International application number:
**PCT/IB2023/056628**

(87) International publication number:
**WO 2024/003742 (04.01.2024 Gazette 2024/01)**

(54) **ELECTROSURGICAL INSTRUMENT FOR APPLYING NON-THERAPEUTIC RF SIGNALS**

ELEKTROCHIRURGISCHES INSTRUMENT ZUR ANWENDUNG NICHT-THERAPEUTISCHER HF-SIGNALE

INSTRUMENT ÉLECTROCHIRURGICAL DESTINÉ À APPLIQUER DES SIGNAUX DE RF NON THÉRAPEUTIQUES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **30.06.2022 US 202217854306**

(43) Date of publication of application:
**15.05.2024 Bulletin 2024/20**

(73) Proprietor: **Cilag GmbH International
6300 Zug (CH)**

(72) Inventors:
• **LAFAY, Eric B.**
**Cincinnati, Ohio 45242 (US)**
• **VANOSDOLL, Madison K.**
**Cincinnati, Ohio 45242 (US)**
• **BORONYAK, Steven M.**
**Cincinnati, Ohio 45242 (US)**
• **PARFETT, Raymond E.**
**Cincinnati, Ohio 45242 (US)**

(74) Representative: **Carpmaels & Ransford LLP
One Southampton Row
London WC1B 5HA (GB)**

(56) References cited:
**EP-A2- 2 335 631      EP-A2- 2 898 846
WO-A1-2022/229872    US-A1- 2017 007 308
US-A1- 2020 315 690   US-A1- 2020 352 628**

**Description**

BACKGROUND

A variety of surgical instruments include a tissue cutting element and one or more elements that transmit radio frequency (RF) energy to tissue (e.g., to coagulate or seal the tissue). An example of such an electrosurgical instrument is the ENSEAL® Tissue Sealing Device by Ethicon Endo-Surgery, Inc., of Cincinnati, Ohio. EP2335631 A2 discloses another electrosurgical instrument according to the prior art. Further examples of such devices and related concepts are disclosed in U.S. Pat. No. 6,500,176 entitled "Electrosurgical Systems and Techniques for Sealing Tissue," issued December 31, 2002; U.S. Pat. No. 8,939,974, entitled "Surgical Instrument Comprising First and Second Drive Systems Actuatable by a Common Trigger Mechanism," issued January 27, 2015; U.S. Pat. No. 8,888,809, entitled "Surgical Instrument with Jaw Member," issued November 18, 2014; U.S. Pat. No. 9,161,803, entitled "Motor Driven Electrosurgical Device with Mechanical and Electrical Feedback," issued October 20, 2015; U.S. Pat. No. 9,877,720, entitled "Control Features for Articulating Surgical Device," issued January 30, 2018; U.S. Pat. No. 9,545,253, entitled "Surgical Instrument with Contained Dual Helix Actuator Assembly," issued January 17, 2017; and U.S. Pat. No. 9,526,565, entitled "Electrosurgical Devices," issued December 27, 2016.

While a variety of surgical instruments have been made and used, it is believed that no one prior to the inventors has made or used the invention described in the appended claims. Methods described here below are useful for the understanding of the invention but are not claimed.

BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:

FIG. 1 depicts a perspective view of an exemplary electrosurgical instrument;

FIG. 2 depicts a perspective view of an exemplary articulation assembly and end effector of the electrosurgical instrument of FIG. 1;

FIG. 3 depicts an exploded view of the articulation assembly and end effector of FIG. 2;

FIG. 4 depicts a perspective view of the end effector that of FIG. 2;

FIG. 5 depicts an exploded perspective view of the end effector of FIG. 2;

FIG. 6 depicts an illustrative schematic diagram of a system in which the electrosurgical instrument of FIG. 1 may be used;

FIG. 7 depicts another illustrative schematic diagram of a system in which the electrosurgical instrument of FIG. 1 may be used;

FIG. 8 depicts an illustrative circuit diagram of a hand switch rectifier circuit;

FIG. 9 depicts an illustrative circuit diagram of a signal conditioning circuit;

FIG. 10 depicts an illustrative circuit diagram of an internal power supply circuit;

FIG. 11 depicts an illustrative circuit diagram of a MOSFET driver and relay circuit;

FIG. 12 depicts an illustrative circuit diagram of voltage and current sensing circuit;

FIG. 13 depicts an exploded perspective view of an example of a cable and a connector assembly;

FIG. 14 depicts an illustrative impedance triangle;

FIG. 15 depicts a set of illustrative example waveforms;

FIG. 16 depicts another set of illustrative example waveforms;

FIG. 17 depicts another illustrative example waveform;

FIG. 18 depicts another illustrative example waveform;

FIG. 19 depicts another set of illustrative example waveforms;

FIG. 20 depicts another illustrative example waveform;

FIG. 21 depicts another illustrative example waveform;

FIG. 22 depicts a set of illustrative graphical examples of impedance magnitude and phase as a factor of frequency for different tissues and materials;

FIG. 23 depicts an illustrative example of cross-correlation of two waveforms;

FIG. 24 depicts another illustrative example of a zero-crossing detection circuit and associated waveforms;

FIG. 25 depicts a graphical representation of the impedance magnitude and the phase angle of a waveform being applied to a tissue as a function of time; and

FIG. 26 depicts various graphs plotting the impedance magnitude and jaw gap vs time.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description explain the principles of the technology; it being understood, however, that this technology is not limited to the precise arrangements shown.

DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

For clarity of disclosure, the terms "proximal" and "distal" are defined herein relative to a surgeon or other operator grasping a surgical instrument having a distal surgical end effector. The term "proximal" refers the position of an element closer to the surgeon or other operator and the term "distal" refers to the position of an element closer to the surgical end effector of the surgical instrument and further away from the surgeon or other operator.

Disclosed here are improved systems and methods for using a surgical instrument to seal and cut tissue. Specifically, the system can use an end effector to capture patient tissue and then test the tissue using a non-therapeutic (i.e., low power) signal to ensure the proper tissue is captured. Once the non-therapeutic signal is applied, received, and analyzed, the system can provide additional detail about the tissue. Assuming the additional details confirm that the captured tissue is the desired tissue, the system can switch operation modes and apply a therapeutic energy signal to the tissue, thereby sealing or cauterizing the tissue.

I. Example of Electrosurgical Instrument

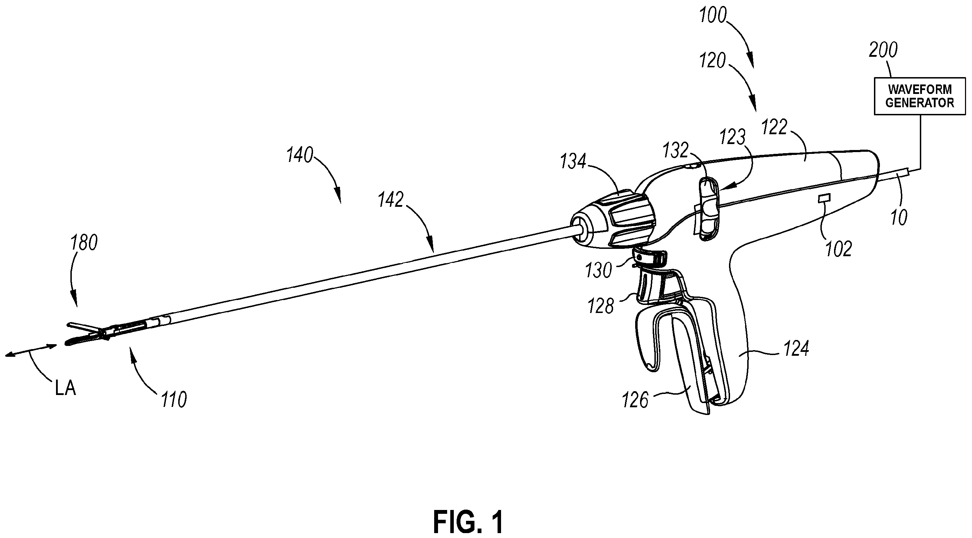

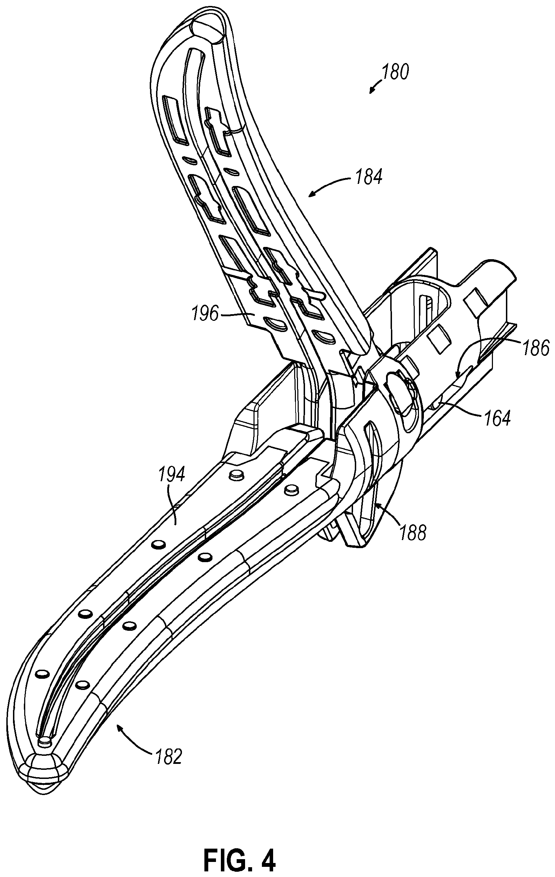

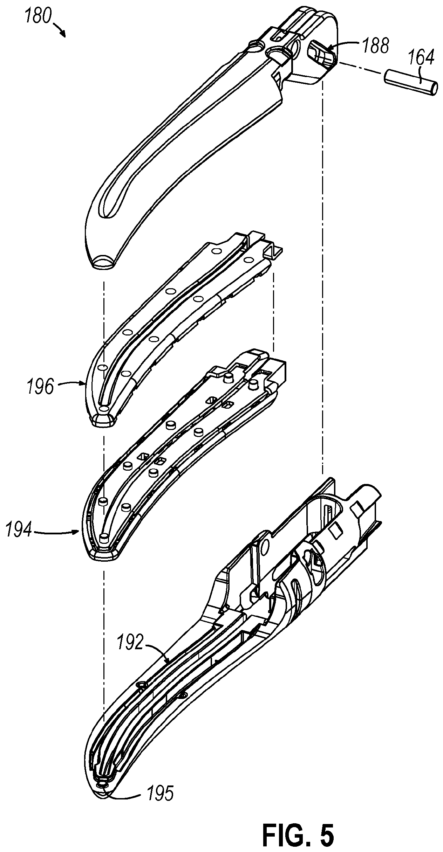

FIGS. 1-5 show an exemplary electrosurgical instrument 100. As best seen in FIG. 1, electrosurgical instrument 100 includes a handle assembly 120, a shaft assembly 140, an articulation assembly 110, and an end effector 180. As will be described in greater detail below, end effector 180 of electrosurgical instrument 100 is operable to grasp, cut, and seal or weld tissue (e.g., a blood vessel, etc.). In this example, end effector 180 is configured to apply a non-therapeutic bipolar radio frequency (RF) energy in order to identify and/or verify that the correct tissue is present in the end effector such that a therapeutic RF energy can be applied to seal or weld tissue. However, it should be understood that electrosurgical instrument 100 may be configured to seal or weld tissue through any other suitable means that would be apparent to one skilled in the art in view of the teachings herein. For example, electrosurgical instrument 100 may be configured to seal or weld tissue via an ultrasonic blade, staples, etc. In the present example, electrosurgical instrument 100 is electrically coupled to a waveform generator 200, which is capable of delivering therapeutic and non-therapeutic energy, via power cable 10.

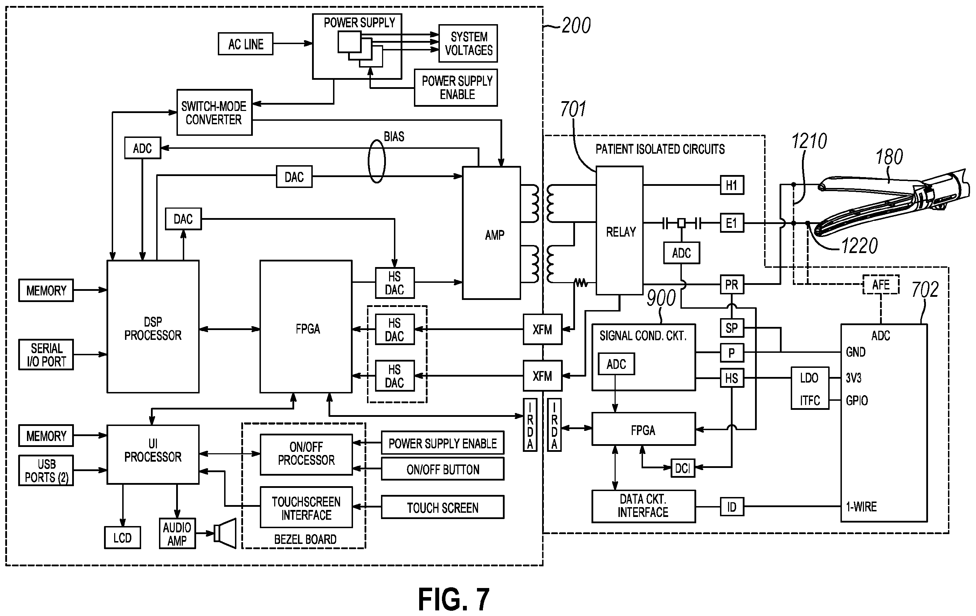

The waveform generator 200 may be configured to provide all or some of the electrical power requirements for use of electrosurgical instrument 100. Any suitable waveform generator 200 may be used as would be apparent to one skilled in the art in view of the teachings herein. By way of non-limiting example, the waveform generator 200 may comprise a GEN04 or GEN11 (shown in FIG. 7) sold by Ethicon, LLC. of Cincinnati, Ohio. In addition, or in the alternative, the waveform generator 200 may be constructed in accordance with at least some of the teachings of U.S. Pat. No. 8,986,302, entitled "Surgical Generator for Ultrasonic and Electrosurgical Devices," issued March 24, 2015. While in the current example, electrosurgical instrument 100 is coupled to a waveform generator 200 via power cable 10, electrosurgical instrument 100 may contain an internal power source or plurality of power sources, such as a battery and/or supercapacitors, to electrically power electrosurgical instrument 100. Of course, any suitable combination of power sources may be utilized to power electrosurgical instrument 100 as would be apparent to one skilled in the art in view of the teaching herein.

Handle assembly 120 is configured to be grasped by an operator with one hand, such that an operator may control and manipulate electrosurgical instrument 100 with a single hand. Although the electrosurgical instrument 100 is primarily described herein as being used by a human user, it should be noted that alternative versions exist in which one or more robotic systems (e.g., a robotic arm) may be used to control and manipulate the electrosurgical instrument 100. Shaft assembly 140 extends distally from handle assembly 120 and connects to articulation assembly 110. Articulation assembly 110 is also connected to a proximal end of end effector 180. As will be described in greater detail below, components of handle assembly 120 are configured to control end effector 180 such that an operator may grasp, cut, and seal or weld tissue. Articulation assembly 110 is configured to deflect end effector 180 from the longitudinal axis (LA) defined by shaft assembly 140.

Handle assembly 120 includes a control unit 102 housed within a body 122, a pistol grip 124, a jaw closure trigger 126, a knife trigger 128, an activation button 130, an articulation control 132, and a knob 134. As will be described in greater detail below, jaw closure trigger 126 may be pivoted toward and away from pistol grip 124 and/or body 122 to open and close jaws 182, 184 of end effector 180 to grasp tissue. Additionally, knife trigger 128 may be pivoted toward and away from pistol grip 124 and/or body 122 to actuate a knife member 176 within the confines of jaws 182, 184 to cut tissue captured between jaws 182, 184. Further, activation button 130 may be pressed to apply radio frequency (RF) energy to tissue via electrode surfaces 194, 196 of jaws 182, 184, respectively. In some versions, electrode surfaces 194, 196 of jaws 182, 184 are in a bifurcation configuration where electrode surfaces 194, 196 move relative to a central axis and nearly equal and opposite to one another.

Body 122 of handle assembly 120 defines an opening 123 through which a portion of articulation control 132 protrudes. Articulation control 132 is rotatably disposed within body 122 such that an operator may rotate the portion of articulation control 132 protruding from opening 123 to rotate the portion of articulation control 132 located within body 122. Rotation of articulation control 132 relative to body 122 will bend articulation section 110 in order to drive deflection of end effector 180 from the longitudinal axis (LA) defined by shaft assembly 140. Articulation control 132 and articulation section 110 may include any suitable features to drive deflection of end effector 180 from the longitudinal axis (LA) defined by shaft assembly 140 as would be apparent to one skilled in the art in view of the teachings herein.

Knob 134 is rotatably disposed on the distal end of body 122 and is configured to rotate end effector 180, articulation assembly 110, and shaft assembly 140 about the longitudinal axis (LA) of shaft assembly 140 relative to handle assembly 120. While in the current example, end effector 180, articulation assembly 110, and shaft assembly 140 are rotated by knob 134, knob 134 may be configured to rotate end effector 180 and articulation assembly 110 relative to selected portions of shaft assembly 140. Knob 134 may include any suitable features to rotate end effector 180, articulation assembly 110, and shaft assembly 140 as would be apparent to one skilled in the art in view of the teachings herein.

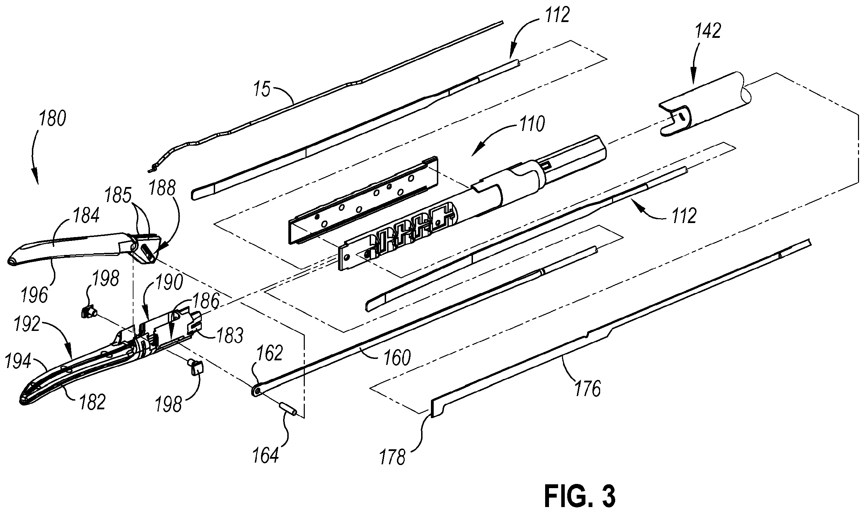

Shaft assembly 140 includes distal portion 142 extending distally from handle assembly 120 and a proximal portion 144 housed within the confines of body 122 of handle assembly 120. Referring now to FIG. 3, shaft assembly 140 houses a jaw closure connector 160 that couples jaw closure trigger 126 with end effector 180. Additionally, shaft assembly 140 houses a portion of knife member extending between distal cutting edge 178 and knife trigger 128. Shaft assembly 140 also houses actuating members 112 that couple articulation assembly 110 with articulation control 132; as well as an electrical connecter 15 that operatively couples electrode surfaces 194, 196 with activation button 130. As will be described in greater detail below, jaw closure connector 160 is configured to translate relative to shaft assembly 140 to open and close jaws 182, 184 of end effector 180; while knife member 176 is coupled to knife trigger 128 of handle assembly 120 to translate distal cutting edge 178 within the confines of end effector 180; and activation button 130 is configured to activate electrode surface 194, 196.

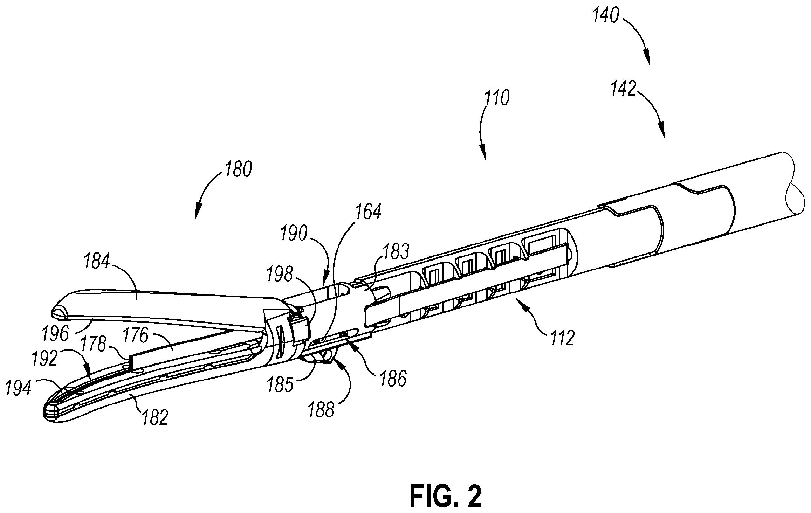

As best seen in FIGS. 2-5, end effector 180 includes lower jaw 182 pivotally coupled with upper jaw 184 via pivot couplings 198. Lower jaw 182 includes a proximal body 183 defining a slot 186, while upper jaw 184 includes proximal arms 185 defining a slot 188. Lower jaw 182 also defines a central channel 190 that is configured to receive proximal arms 185 of upper jaw 184, portions of knife member 176, jaw closure connecter 160, and pin 164. Slots 186, 188 each slidably receive pin 164, which is attached to a distal coupling portion 162 of jaw closure connector 160. Additionally, lower jaw 182 includes a force sensor 195 located at a distal tip of lower jaw 182, though force sensor 195 may alternatively be positioned at any other suitable location. Force sensor 195 may be in communication with control unit 102. Force sensor 195 may be configured to measure the closure force generated by pivoting jaws 182, 184 into a closed configuration in accordance with the description herein. Additionally, force sensor 195 may communicate this data to control unit 102. Any suitable components may be used for force sensor 195 as would be apparent to one skilled in art in view of the teachings herein. For example, force sensor 195 may take the form of a strain gauge. In some variations, end effector 180 includes more than one force sensor.

While in the current example, a force sensor 195 is incorporated into instrument 100 and is in communication with control unit 102, any other suitable sensors or feedback mechanisms may be additionally or alternatively incorporated into instrument 100 while in communication with control unit 102 as would be apparent to one skilled in the art in view of the teachings herein. For instance, an articulation sensor or feedback mechanism may be incorporated into instrument 100, where the articulation sensor communicates signals to control unit 102 indicative of the degree end effector 180 is deflected from the longitudinal axis (LA) by articulation control 132 and articulation section 110.

As will be described in greater detail below, jaw closure connector 160 is operable to translate within central channel 190 of lower jaw 182. Translation of jaw closure connector 160 drives pin 164. As will also be described in greater detail below, with pin 164 being located within both slots 186, 188, and with slots 186, 188 being angled relative to each other, pin 164 cams against proximal arms 185 to pivot upper jaw 184 toward and away from lower jaw 182 about pivot couplings 198. Therefore, upper jaw 184 is configured to pivot toward and away from lower jaw 182 about pivot couplings 198 to grasp tissue.

The term "pivot" does not necessarily require rotation about a fixed axis and may include rotation about an axis that moves relative to end effector 180. Therefore, the axis at which upper jaw 184 pivots about lower jaw 182 may translate relative to both upper jaw 184 and lower jaw 182. Any suitable translation of the pivot axis may be used as would be apparent to one skilled in the art in view of the teachings herein.

Lower jaw 182 and upper jaw 184 also define a knife pathway 192. Knife pathway 192 is configured to slidably receive knife member 176, such that knife member 176 may be retracted, and advanced, to cut tissue captured between jaws 182, 184.

Lower jaw 182 and upper jaw 184 each comprise a respective electrode surface 194, 196. The power source may provide RF energy to electrode surfaces 194, 196 via electrical coupling 15 that extends through handle assembly 120, shaft assembly 140, articulation assembly 110, and electrically couples with one or both of electrode surfaces 194, 196. Electrical coupling 15 may selectively activate electrode surfaces 194, 196 in response to an operator pressing activation button 130. In some instances, control unit 102 may couple electrical coupling 15 with activation button 130, such that control unit 102 activates electrode surfaces 194, 196 in response to operator pressing activation button 130. Control unit 102 may have any suitable components in order to perform suitable functions as would be apparent to one skilled in the art in view of the teachings herein. For instance, control unit 102 may have a processor, memory unit, suitable circuitry, etc. Examples of features and functionalities that may be incorporated into control unit 102 will be described in greater detail below.

As described above, jaw closure trigger 126 may be pivoted toward and away from pistol grip 124 and/or body 122 to open and close jaws 182, 184 of end effector 180 to grasp tissue. In particular, as will be described in greater detail below, pivoting jaw closure trigger 126 toward pistol grip 124 may proximally actuate jaw closure connector 160 and pin 164, which in turn cams against slots 188 of proximal arms 185 of upper jaw 184, thereby rotating upper jaw 184 about pivot couplings 198 toward lower jaw 182 such that jaws 182, 184 achieve a closed configuration.

In some versions, knife trigger 128 may be pivoted toward and away from body 122 and/or pistol grip 124 to actuate knife member 176 within knife pathway 192 of jaws 182, 184 to cut tissue captured between jaws 182, 184. In particular, handle assembly 120 further includes a knife coupling body 174 that is slidably coupled along proximal portion 144 of shaft assembly 140. Knife coupling body 174 is coupled with knife member 176 such that translation of knife coupling body 174 relative to proximal portion 144 of shaft assembly 140 translates knife member 176 relative to shaft assembly 140.

In another version, knife coupling body 174 may be coupled to a knife actuation assembly such that as knife trigger 128 pivots toward body 122 and/or pistol grip 124, knife actuation assembly 168 drives knife coupling body 174 distally, thereby driving knife member 176 distally within knife pathway 192. Because knife coupling body 174 is coupled to knife member 176, knife member 176 translates distally within shaft assembly 140, articulation section 110, and within knife pathway 192 of end effector 180. Knife member 176 includes distal cutting edge 178 that is configured to sever tissue captured between jaws 182, 184. Therefore, pivoting knife trigger 128 causes knife member 176 to actuate within knife pathway 192 of end effector 180 to sever tissue captured between jaws 182, 184.

With distal cutting edge 178 of knife member 176 actuated to the advance position, an operator may press activation button 130 to selectively activate electrode surfaces 194, 196 of jaws 182, 184 to weld/seal severed tissue that is captured between jaws 182, 184. It should be understood that the operator may also press activation button 130 to selectively activate electrode surfaces 194, 196 of jaws 182, 184 at any suitable time during exemplary use. Therefore, the operator may also press activation button 130 while knife member 176 is retracted. Next, the operator may release jaw closure trigger 128 such that jaws 182, 184 pivot into the opened configuration, releasing tissue.

II. Description of Overall System and Specific Circuitry

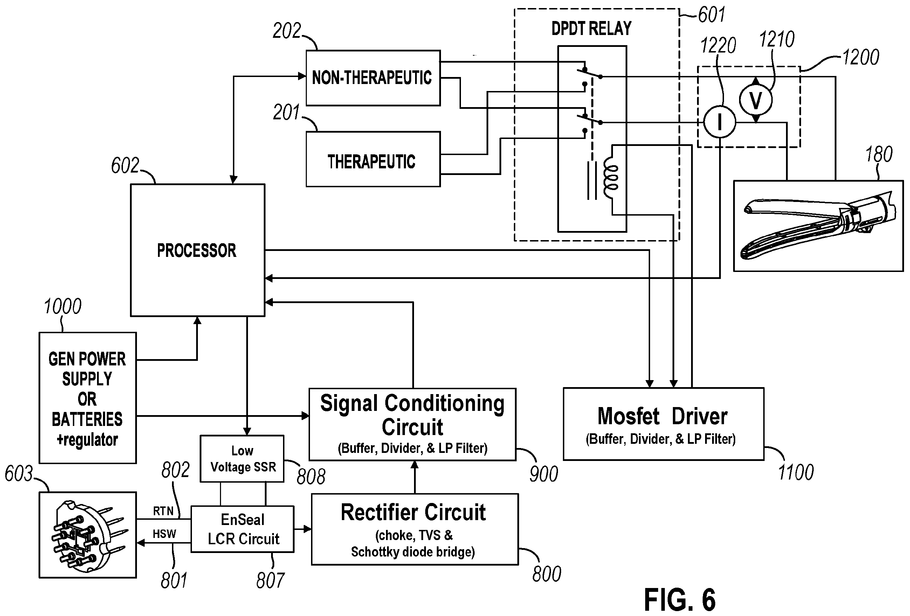

An illustrative schematic of an example system is shown in FIG. 6. As discussed herein, the electrosurgical instrument 100 may include some form of a control unit (e.g., control unit 102 in handle assembly 120 and/or control unit features in waveform generator 200). In some versions, and as discussed herein, the control unit may enable the electrosurgical instrument 100 to apply two different types (e.g., therapeutic and non-therapeutic) of a RF signal. In some versions, which will be discussed in detail herein, a switching system (e.g., a switching relay or the like) 601 may allow the system to switch or alternate between therapeutic and non-therapeutic signals. In some versions, the therapeutic and non-therapeutic signals may be generated by single waveform generator 200 that contains therapeutic 201 and non-therapeutic 202 signal generators. However, in some alternative versions, the therapeutic 201 and non-therapeutic 202 signal generators may be standalone devices.

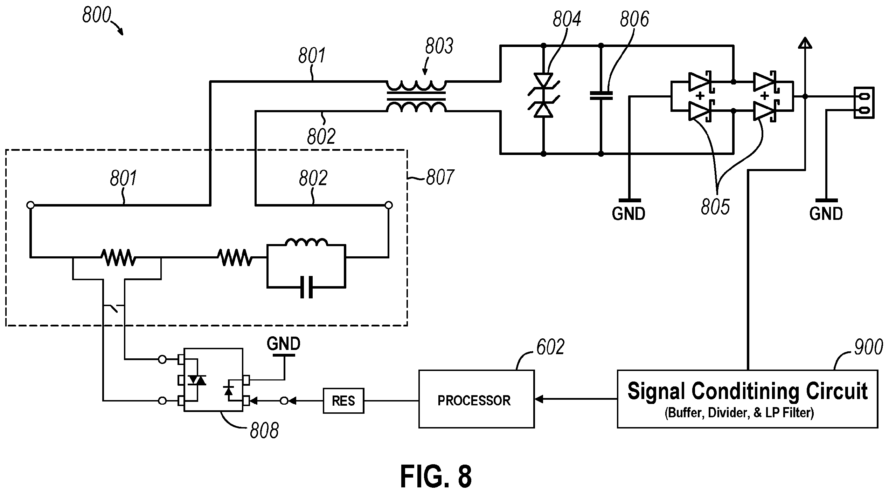

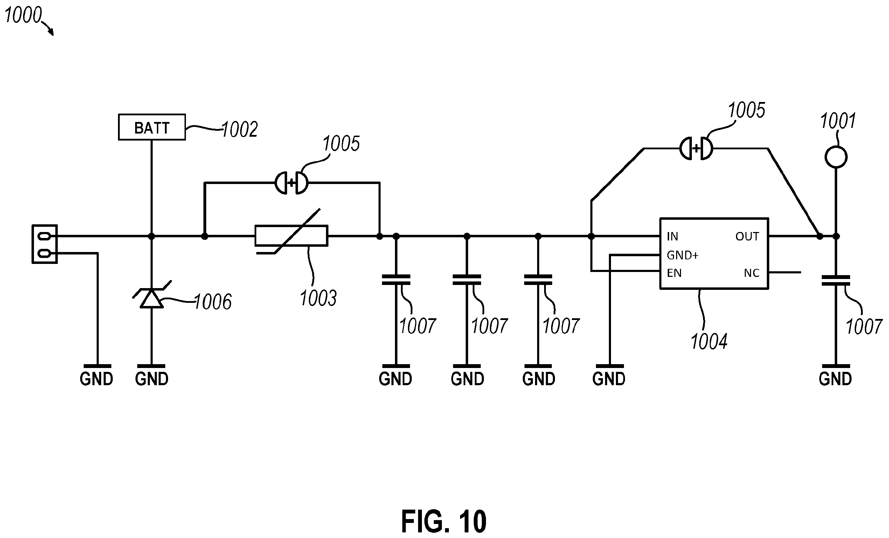

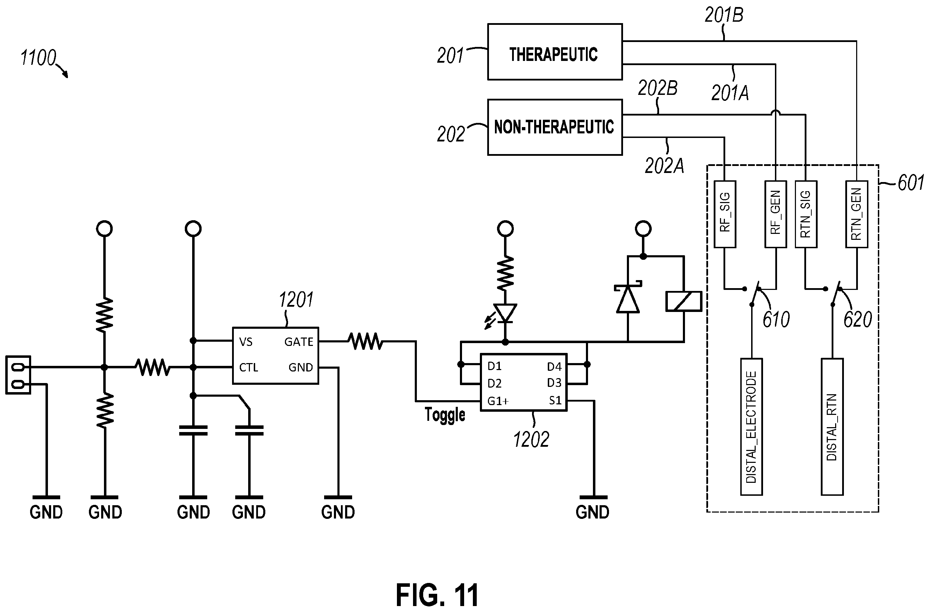

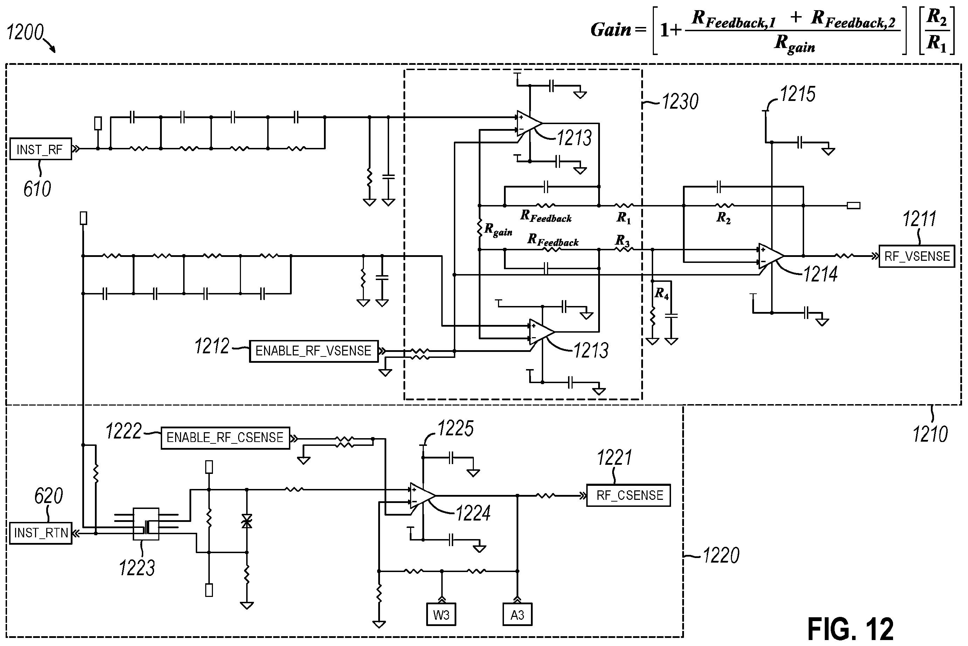

As will be described in more detail here, the process for determining which signal (e.g., therapeutic v. non-therapeutic) the switching system 601 selects may be based on various factors and determinations. In some versions, a processor 602 may be used to facilitate with the signal selection. As used herein, the term "processor" shall be understood to include a microprocessor, a micro controller, a field programmable gate array (FPGA) device, and/or any other suitable kind(s) of hardware configured to process electrical signals. In further versions, and as shown, the system may also include: a hand switch rectifier circuit 800 (shown in detail in FIG. 8, which may include a low voltage solid state relay 808 and a sealing circuit 807 configured to connect to a drive signal 801 and a return signal 802 via the pin connector 603); a signal conditioning circuit 900 (shown in detail in FIG. 7); an internal power supply 1000 (shown in detail in FIG. 10); a MOSFET driver circuit 1100 (shown in detail in FIG. 11); a voltage sensing circuit 1210 (shown in detail in FIG. 12); and a current sensing circuit 1220 (shown in detail in FIG. 12). It should be understood that the circuits shown and discussed herein are shown in detail solely for illustrative purposes, and no circuits or circuit diagrams should be considered limiting or restrictive to any version disclosed here. Stated differently, one of skilled in the art would understand that alternate and/or modified circuits may exist, both now and in the future, and those circuits may be used to facilitate certain portions of the design disclosed herein.

By way of non-limiting example, FIG. 7 shows an exemplary circuit diagram that may be employed in a GEN 11 waveform generator sold by Ethicon, LLC. Thus, in some versions, and as shown in FIG. 7, the system may include a waveform generator 200 that can provide both therapeutic 202 and non-therapeutic 201 waveforms to the switching system 701, which in turn selects which waveform to pass onto end effector 180. Similar to the version discussed with reference to FIG. 6, the circuit may include a processor 702, and various other circuits (e.g., the signal conditioning circuit 900).

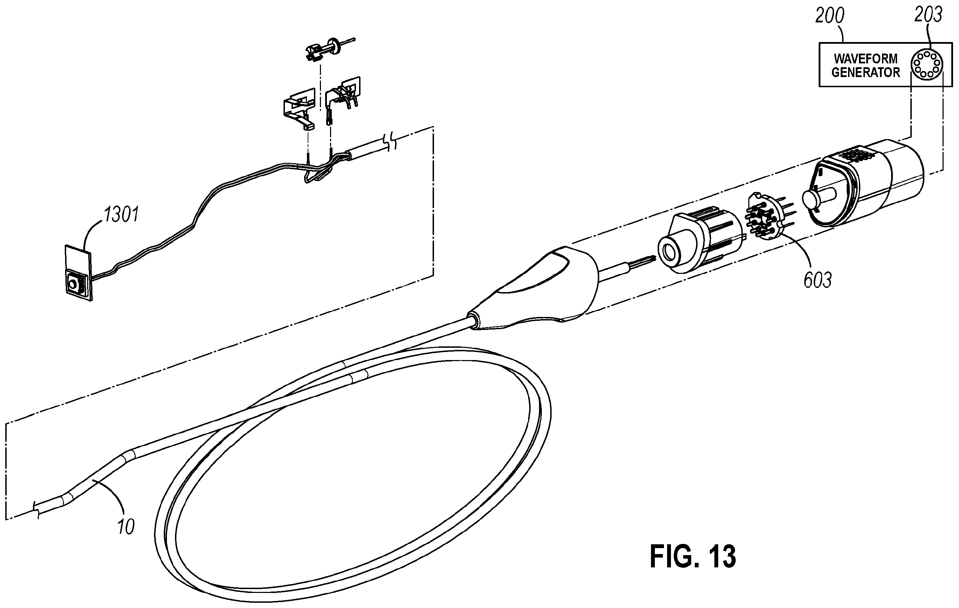

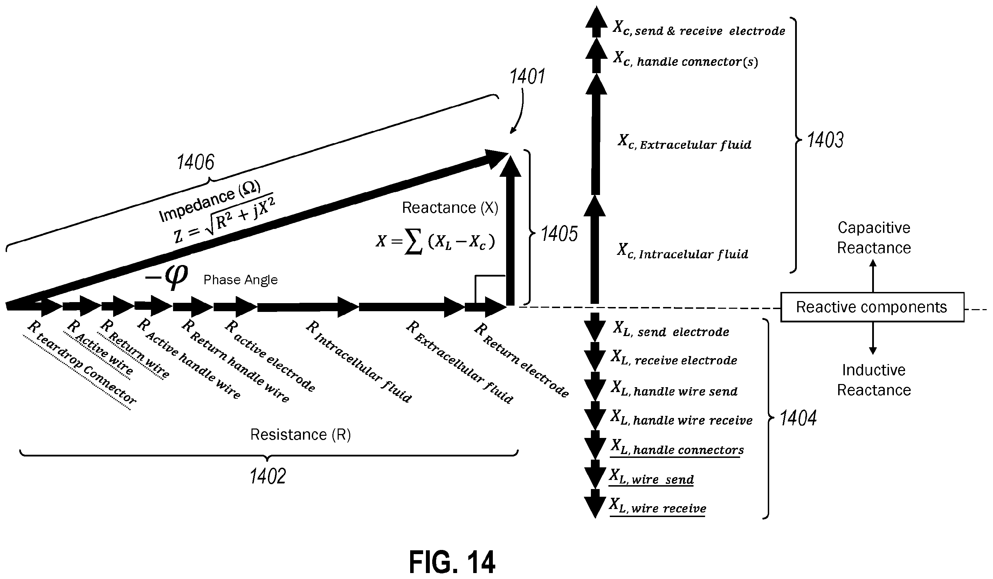

As shown in FIG. 6, and again below in FIG. 14, the handle assembly 120 may be connected to a waveform generator 200 via cable 10. In some versions, the system may be adapted to operate on legacy equipment. For example, various existing therapeutic systems may utilize a 9-pin connector (e.g., 603), which has at least one available pin to allow for transmission of non-therapeutic energy. Thus, as shown in FIGS. 6 and 13, the system may utilize a pinned connector 603 to pass the various signals between the electrosurgical instrument 100 and the wave generator 200.

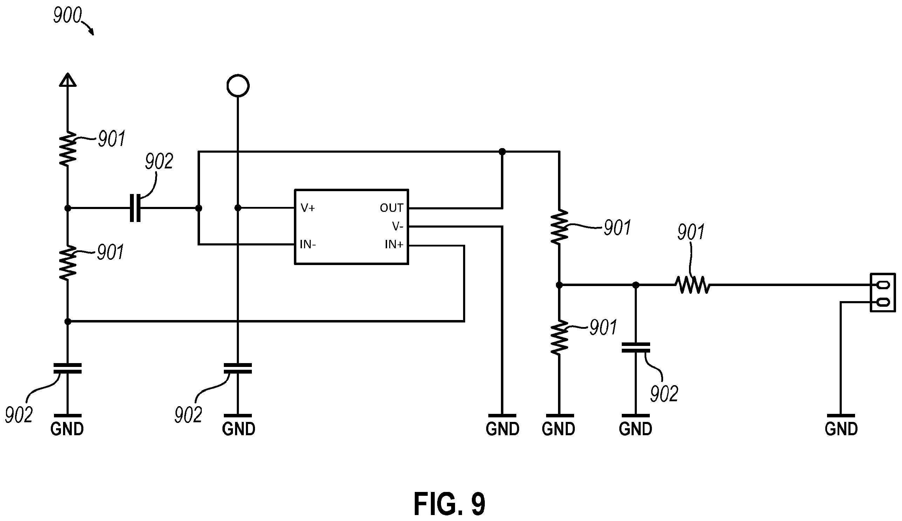

In addition to operation on legacy waveform generation equipment, the systems and methods described herein may also be used on legacy electrosurgical instruments (e.g., electrosurgical instruments similar to that shown in FIG. 1, but without the circuitry shown in FIGS. 8-13). Stated differently, some implementations may exist in which an external housing (e.g., disposable or reusable) contains the circuitry, and thus the functionality, described with reference to FIGS. 8-13. In some implementations, the external housing may be mounted (e.g., operatively coupled) to the handle assembly 120 of the electrosurgical instrument. Alternative implementations may exist in which the external housing is coupled with an alternative device or location, such as, for example, one of the waveform generators, a patient bed, surgical tool, or other object within the surgical theater. Moreover, the components providing the functionality of the components described with reference to FIGS. 8-13 need not be contained within a dedicated external housing. Such components may be integrated into another housing with other components. For instance, such components may be integrated into a housing of a waveform generator, etc. FIGS. 8-13 show detailed example circuit diagrams of various forms that may be taken by the circuits more generally shown in FIG. 6. For instance, FIG. 8 shows an example of a form that rectifier circuit 800 may take. As shown in FIG. 8, a rectifier circuit 800 receives a hand switch drive signal 801 and/or a hand switch return signal 802 (e.g., from activation button 130 or similar trigger device). In some versions, the rectifier circuit may include or connect to solid-state relay 808, and/or a sealing circuit 807. Similar to the system shown in FIG. 6, the rectifier circuit 800 may connect to the signal conditioning circuit 900 and a processor 602. The rectifier circuit 800 may further include a choke (e.g., a common mode choke) or filter 803, that receives signals 801/802 from the handle assembly 120. The drive signal 801 and/or return signal 802 may then pass the electrostatic discharge diodes 804 (or transient voltage suppression (TVS) diodes) and capacitor 806 before being rectified (e.g., using a fast Schottky diode 805 bridge-based rectifier) and passed to the next component, such as, the signal conditioning circuit 900 as shown in FIG. 6. FIG. 9 shows an example of a form that signal conditioning circuit 900 may take.

Once rectified, the signal may then pass to the signal conditioner 900 shown in FIG. 9. In some versions, the conditioner circuit 900 may include one or more resistors 901, one or more capacitors 902, and an operational amplifier (op amp) 903. In some variations, one or more inductors (not shown) are included, in addition to or in lieu of including capacitors 902. In a further version, the op amp 903 may be a very high impedance input op amp configured as a buffer and paired with a passive voltage divider and passive low pass filter. In some such scenarios, the buffer op amp of this circuit may serve to restrict all incoming electromagnetic interference and capacitive coupling that the waveform generator 200 may produce. The passive voltage divider and passive filter may attenuate the buffered signal and smooth out transients from, the incoming signal. The resulting signal is presented to the processor 602, which may use the signal to decide when to use therapeutic vs non-therapeutic energy delivery. Once the hand switch drive signal 801 and/or a hand switch return signal 802 is conditioned (i.e., passes through the circuit 900), it may then be passed to the microprocessor 602/702 for evaluation.

FIG. 10 shows an example of a form that may be taken by power supply circuit 1000 of FIG. 6. In some versions, and as shown in FIG. 10, the power supply may have an external power source 1001 and/or a battery power source 1002. In addition to the two power sources 1001/1002, the power supply circuit 1000 may include a thermistor 1003 and a voltage regulator 1004, each with their own jumper 1005 such that they can be automatically bypassed. In a further version, the power supply circuit may also include one or more diodes 1005, and one or more capacitors 1007.

FIG. 11 shows an example of a form that may be taken by MOSFET relay driver circuit 1100 of FIG. 6. As shown in FIG. 11, the MOSFET relay driver circuit may have one or more MOSFET 1201 to drive one or more MOSFETs 1202 via the "Toggle" input. The MOSFET 1202 may then be used, as shown in FIG. 6, to change the state of the switching system 601. The MOSFET relay driver circuit 1100 is, in some versions, attached to a dual position dual throw (DPDT) electro-mechanical relay 601. Some variations may include multiple solid state relays, mechanical switches, and/or other components in addition to or in lieu of including DPDT relay 601. In some versions, the processor 602 may select when to toggle energy from therapeutic and non-therapeutic energy delivery based on hand switch signals. In a further version, a less than 12ms delay may be required to toggle due to the electro-mechanical action of charging and discharging the coil contained in relay mechanism. Thus, in some versions, a group of 4 solid state relays may be used to achieve the same results, but with a much faster response time due to the lack of mechanical redundancy.

A graphical illustration of the switching circuit 601 of FIG. 6 is shown bounded by a dashed box in FIG. 11. In some versions, and as shown in FIG. 11, the electrosurgical instrument 100 may receive a waveform (e.g., therapeutic or non-therapeutic) via the send path 610 and return the waveform via the return path 620. Accordingly, in some versions, the therapeutic waveform generator 201 may output a waveform to one side 201A of the send relay 610 and receive the resultant waveform (i.e., the waveform after it passes through the electrodes of the end effector 180) from one side 201B of the receive relay 620. Similarly, the non-therapeutic waveform generator 202 may output a waveform to one side 202A of the send relay 610 and receive the resultant waveform from one side 202B of the receive relay 620. Examples of such operation are described in greater detail below with reference to FIGS. 14-15.

In some versions, the switching system 601 may include a double-pole double-throw (DPDT) relay, which may have two sets of switches or positions, where each switch has with two options contacts or throws. Each relay position may have a connection to a therapeutic energy electrode or return and non-therapeutic electrode or return. Each position may have a normally open (NO) or normally closed (NC) throw when the relay coil is non-energized. In certain versions, the switching system 601 (e.g., switching relay) may have non-therapeutic energy delivery set to normally closed whenever the user is not depressing the hand switch to allow for bio-impedance sensing. However, once actuation of the hand-switch (e.g., activation button 130) has taken place, the switching system 601 may throw to the normally open (NO) contact and start therapeutic energy delivery. In some versions, the switching system 601 may be located in the handle 120, while in other versions, it may be in the generator 200 itself.

As discussed herein, with reference to FIG. 6, the system may have a voltage and current detection system 1200. Referring now to FIG. 12, in some versions, the detection system 1200 may have a voltage sensing component 1210 and a current sensation component 1220 to detect the voltage drop between send relay 610 and the receive relay 620 and the current returning to the waveform generator (e.g., 201 or 202) via the receive relay 620. In some versions, and as shown, the sensing circuits may receive a signal from the send relay 610 and pass the signal back to the voltage and current detection system 1200 via the return relay 620. In a further version, the voltage sense circuit 1210 may include an output 1211 that provides the measured voltage to the processor 602. The voltage sense circuit 1210 may also have a toggle switch 1212 that can be used to enable or disable the voltage sense circuit 1210. In an additional version, the current sense circuit 1220 may include an output 1221 that provides the measured current to the processor 602. The current sense circuit 1220 may also have a toggle switch 1222 that can be used to enable or disable the current sense circuit 1220.

In some versions, and as shown, the voltage sense circuit 1210 may be constructed of two operational amplifiers 1213 operating as an inverter 1230 and a summation amplifier 1214 placed in a series configuration with the inverter. As shown, the input inverting amplifiers 1213 are designed to attenuate and invert the stimulus signal (e.g., the send signal received from the send relay 610) based on the ratio of the feedback resistors. The result of the inverting operational amplifier will be an attenuated or lower voltage signal. In some versions, this lower voltage signal may then be shifted to a signal capable of being sensed by a microprocessor (such as processor 602 shown in FIG. 6) by a second stage non-inverting summing amplifier 1214. The input of the non-inverting summing amplifier 1214 is a combination of the inverting operational amp output a tune-able DC voltage 1215. The DC voltage 1215 may be supplied by either a digital-to-analog converter (not shown), a digital potentiometer (not shown), or voltage reference integrated circuit (not shown). The gain of the non-inverting summing amplifier is one plus the ratio of feedback resistors, as shown in the following equation:

$$\text{Equation 1:} \quad Gain = \left[1 + \frac{R_{Feedback,1} + R_{Feedback,2}}{R_{gain}}\right]\left[\frac{R_2}{R_1}\right]$$

The current sense circuit 1220 may, in some versions, be constructed of low an impedance sense resistor tied to a high common mode differential instrument amplifier circuit 1223. The instrument amplifier 1223 will convert the differential sense signal into a single ended low voltage signal. The signal gain is the common instrument op amp gain of (1+(R11+R12)/R10) multiplied by (R16/R14). This low voltage signal is then processed by a second stage non-inverting summing amplifier 1224. The input of the non-inverting summing amplifier is a combination of the instrument operational amplifier 1223 output and the tune-able DC voltage 1225 supplied by a digital-to-analog converter (not shown), a digital potentiometer (not shown). or voltage reference integrated circuit (not shown). The gain of the non-inverting summing amplifier is one plus the ratio of R20/R19.

Referring now to FIG. 13, an example waveform generator 200 is shown as having a connection point 203 that is capable of accepting the pinned connected (603 in FIG. 6), discussed herein. The pinned connector 603 is then connected to a plurality of wires/cables that pass through the power cable 10 that connects to electrosurgical instrument 100. In some versions, the instrument 100 may include a connection port (not shown) that allows for the connection and disconnection of a power adapter 1301.

III. Description of System Operation and Capability

The systems discussed herein and shown in FIGS. 1-13 provide for an electrosurgical instrument 100 that is configured to clamp tissue using an end effector 180. Once securely clamped, electrodes (e.g., the electrodes on electrode surface 194 and/or 196) in the end effector 180 apply a non-therapeutic (i.e., low voltage) waveform to the tissue; and sensor devices (e.g., 1200) measure the returning waveform to calculate and measure the impedance of the tissue. More specifically, the system via one or more sub-circuits will provide non-therapeutic energy to the extracellular and intracellular fluid present within a given (e.g., clamped) region of tissue to determine a phase and a magnitude of the impedance of the tissue within jaws 182/184. The processor 602 may then relay information associated with the tissue, such as, for example, tissue type, tissue phase, tissue margin, and the like. Using this associated information, the system can not only verify that the proper tissue is clamped between the jaws 182/184 but can also determine if any non-tissue material is present between the jaws, and/or if a proper seal has been created after applying the therapeutic RF.

FIG. 14 shows an illustrative impedance triangle 1401. As would be understood by one skilled in the art, human tissues may tend to be capacitive in nature, while wires, tool, staples, implants, etc. may tend to be inductive in nature. Thus, as can be seen by the illustrative impendence triangle 1401, the "resistance" of each object in the circuit is measured 1402 using the waveform and sensors 1200. The system can also determine the "capacitive reactance" of each object in the circuit 1403 and the inductive reactance of each object in the circuit 1404. As discussed above, and clearly shown in FIG. 14, the send and receive electrodes (e.g., electrodes on electrode surfaces 194, 196), the send and receive handle wires (e.g., 610 and 620), the handle connector (e.g., 1301), and the send and receive wires (e.g., included in power cable 10) all have inductive reactance 1404. Additionally, the send and receive electrodes (e.g., electrodes on electrode surfaces 194, 196), the handle connector (e.g., 1301), the extracellular fluid, and the intracellular fluid all have capacitive reactance 1403. The "reactance" 1405 can then be calculated by determining the difference between the capacitive reactance and the inductive reactance using:

$$\text{Equation 2: } X = \sum(X_L - X_C).$$

As shown in FIG. 14, the "impendence" 1406 can then be determined using:

$$\text{Equation 3: } Z = \sqrt{R^2 + jX^2}$$

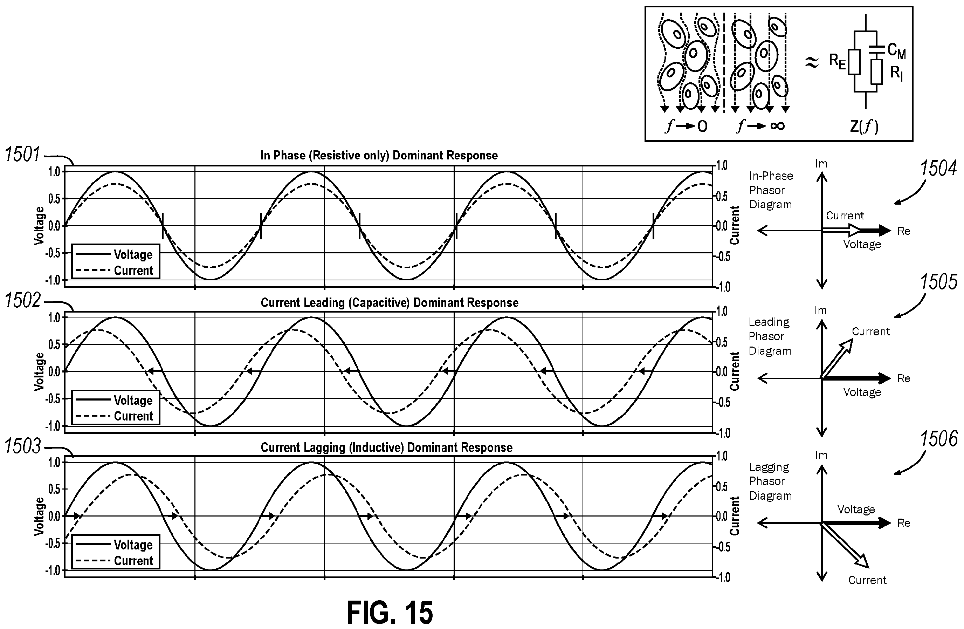

FIG. 15 shows a set of illustrative example waveforms. As would be understood by one skilled in the art, if a circuit only contains resistive items, the current and voltage will remain in phase such as shown in graph 1501 and phasor diagram 1504. Alternatively, if the circuit has capacitive objects, or more capacitive than inductive, the voltage wave will lead the current wave such as shown in graph 1502 and phasor diagram 1505. Finally, if the circuit has inductive objects, or more inductive objects than capacitive objects, the voltage will lag behind the current, such as shown in graph 1503 and phasor diagram 1506.

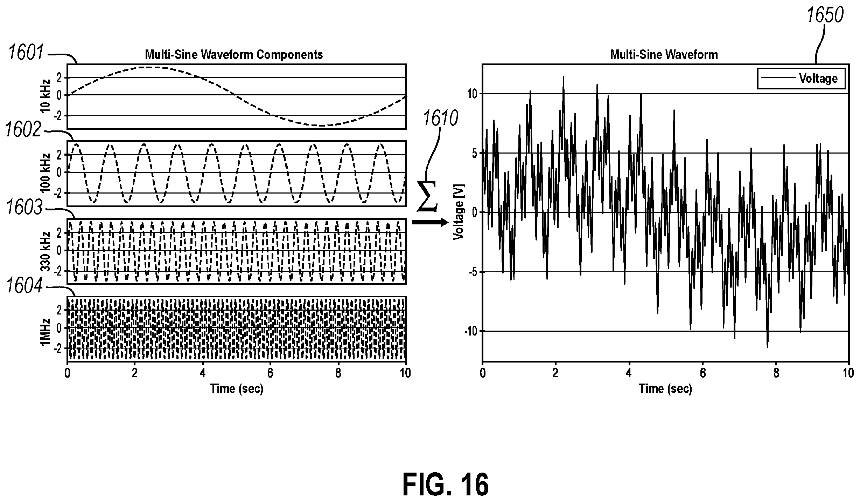

As discussed herein, the system may pass a non-therapeutic waveform through a portion of patient tissue to help identify the type of tissue as well as any foreign objects. Thus, in some versions, the system may pass waveforms of varying frequency (e.g., in series and/or parallel) to improve the accuracy of the determination. Accordingly, in some versions, and as shown in FIG. 16, multiple waveforms of various frequencies may be added or summed together 1610 to create a muti-sine waveform 1650. My way of non-limiting example, a 10kHz sine wave 1601 may be combined with a 100kHz sine wave 1602, a 330kHz sine wave 1603 and a 1MHz sine wave 1604 may be combined to create the multi-sine wave 1650.

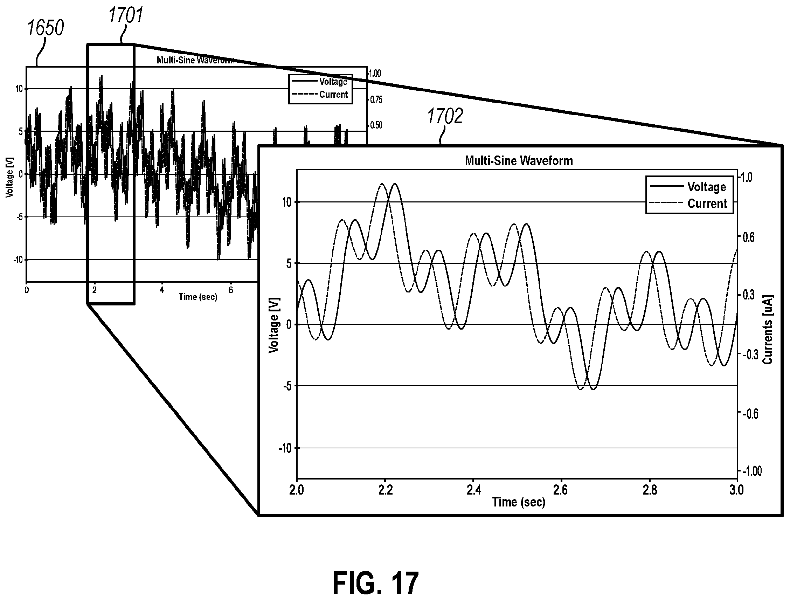

Referring now to FIG. 17, the multi-sine waveform 1650 may be sampled or windowed 1701. In some versions, such as those that require the use of Fast Fourier Transforms (FFT), the windowing or sampling may be as small as a single period for the lower frequency waveform. As shown in graph 1702, the voltage of the multi-sine waveform is leading the current and thus indicates a capacitive circuit (e.g., likely tissue). In an alternative version, the system may apply a series of burst waveforms having different frequencies.

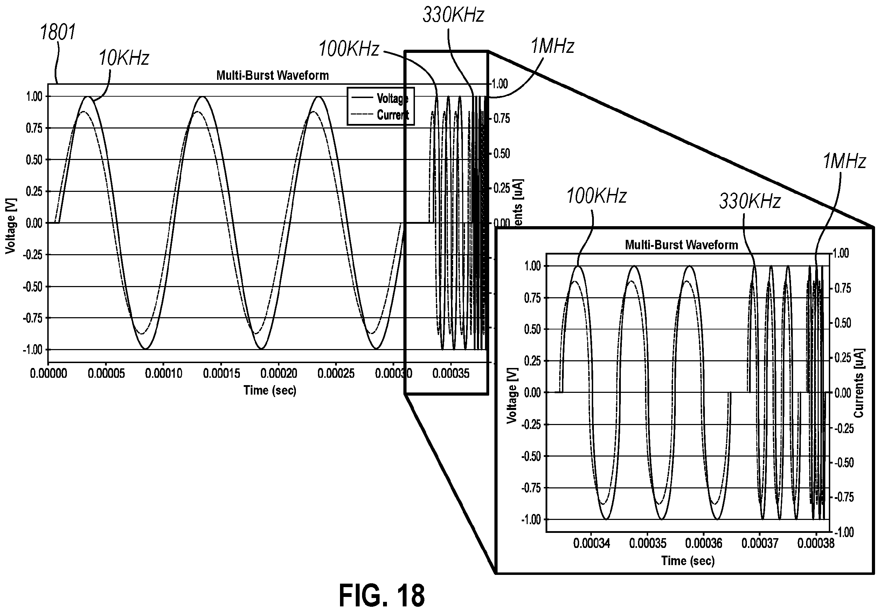

Referring now to FIG. 18, a burst waveform, including a brief delay between frequencies, is shown in graph 1801. In some versions, and as shown, the system may output a burst waveform that is a sine wave, while in other versions, the wave may be a square, triangle, ramp, pulse, pseudorandom binary sequence (PRBS), or arbitrary waveform. In some versions, the pause between waveforms can be evaluated in order to determine a "rebounding" time. The rebounding time may be used to help identify tissue types by evaluating how long certain tissues take to allow the waveform and any residual energy to dissipate from the tissue.

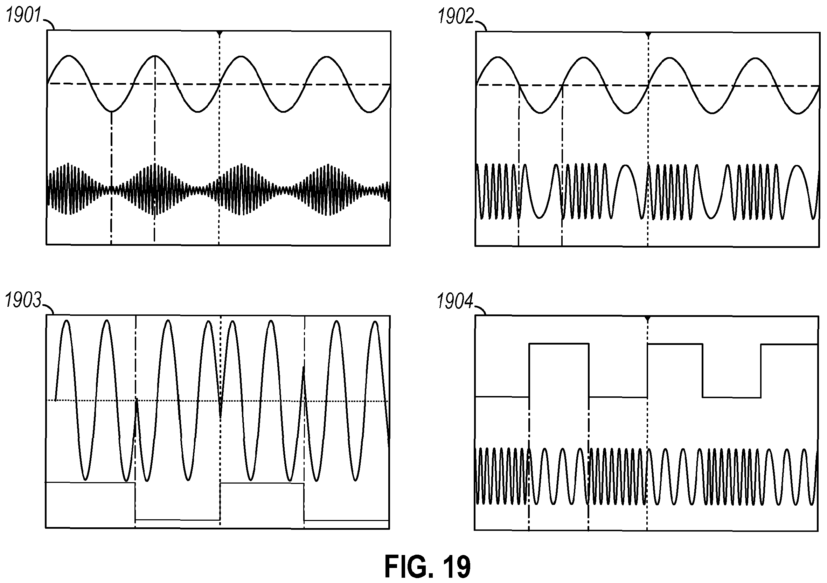

FIG. 19 shows various alternative burst versions. Specifically, in one version, amplitude modulation (AM) 1901 may be used; while in another version, frequency modulation (FM) 1902. Other versions may use phase modulation (PM) 1903 and/or frequency-shift keying (FSK) modulation 1904. Due to the fact that all of the modulation options shown in FIG. 19 involve a shift of some type, they may all be evaluated in a similar manner.

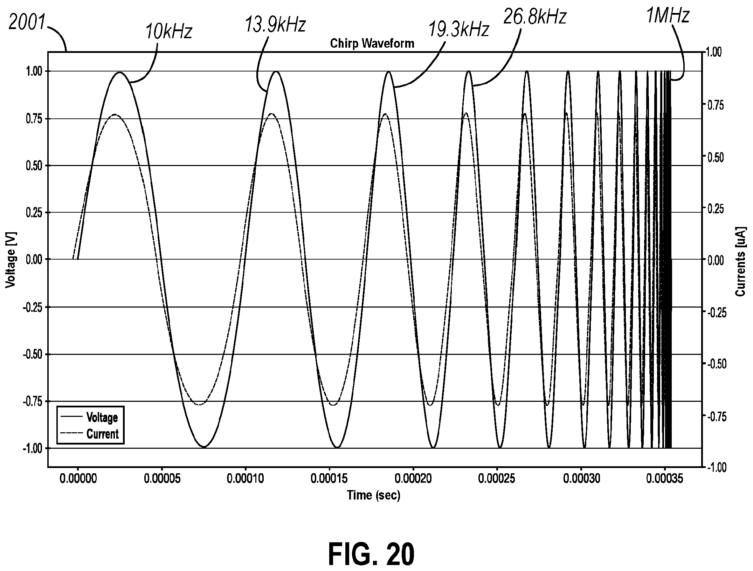

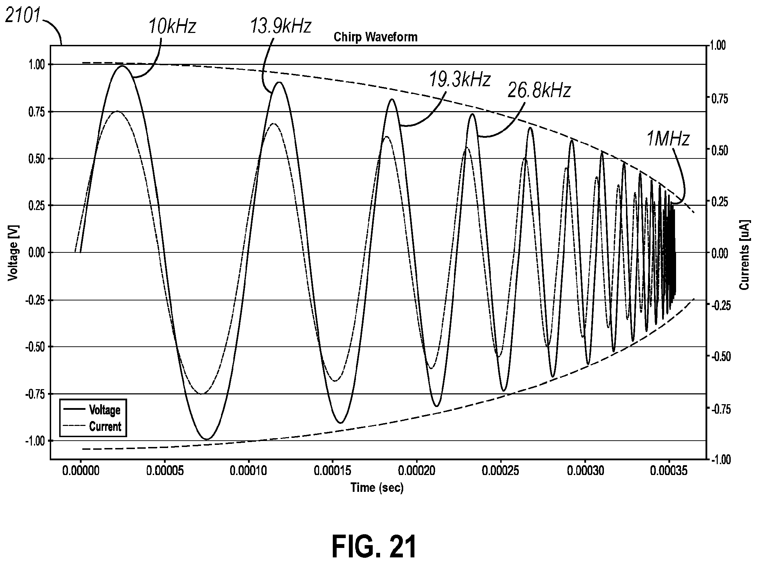

In a further version, a "chirp" function can be used, such as shown in FIG. 20. As would be understood by one skilled in the art, a chirp wave can be an "up-chirp" (i.e., the frequency increases) or a "down-chirp" (i.e., the frequency decreases). Thus, stated differently, a chirp function is essentially an advanced form of FM 1902. The chirp function shown in graph 2001 shows a chirp waveform with increasing frequency (e.g., 10kHz, 13.2kHz, 19.3kHz, 26.8kHz, and 1Mhz). FIG. 21 shows a chirp function with the same frequencies as shown in FIG. 20, but with a decreasing amplitude 2101.

IV. Analysis of Waveforms

The following discussion provides illustrative examples regarding how processor 602 may process feedback signals received from the tissue, via the electrode surfaces 194/196, in response to non-therapeutic and/or therapeutic signals that are applied to the tissue via the electrode surfaces 194/196. For example, if it is determined that a non-tissue object was clamped between the jaws 182/184, the processor 602 may alert the user (e.g., via a visual indicator on the electrosurgical instrument 100, a visual indicator in a display device, an auditory notification, a haptic notification, and the like) and/or lockout the ability to apply RF voltage to the end effector 180.

As discussed herein, Fast Fourier Transforms (FFT) can be one method of analyzing the waveforms to determine a phase and/or impedance. As should be understood by one skilled in the art, FFT functions can map time-domain functions into frequency-domain representations. Generally, FFT is derived from the Fourier transform equation, which is:

$$\text{Equation 4: } X(f) = F\{x(t)\} = \int_{-\infty}^{\infty} x(t)e^{-j2\pi ft}\, dt$$

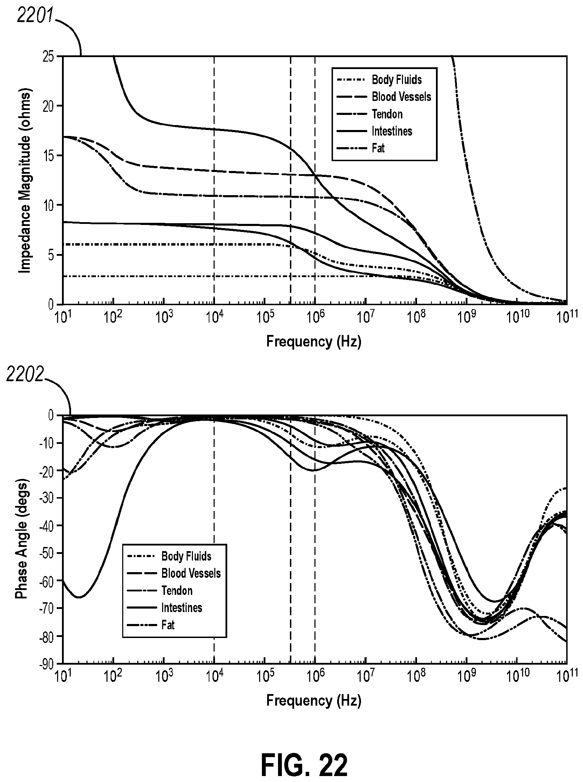

where x(t) is the time domain signal, X(f) is the FFT, and ft is the frequency to analyze. Once the waveform or multi-waveform has been transformed to the frequency domain, the system can evaluate and determine, based on known characteristics, the frequency, impendence and/or phase angle. For example, FIG. 22 shows two graphs, including one graph plotting frequency v. impedance 2201 and one graph plotting frequency v. phase angle 2202. As can be seen in graphs 2201 and 2202, various tissue types (e.g., body fluids, blood vessels, tendons, intestines, and fat) each have different values based on the frequency applied. In some versions, trends may become apparent in the data. Thus, in some versions, a system may include an artificial intelligence module that can train on a data set and learn how to adapt and predict the type of tissue based on the frequency and its associated impedance and/or phase angle. In other versions, the tissue types may simply be values that are referenced and/or searched (e.g., a database) and correlated to the sensed information (e.g., the voltage and current sensed by sensor 1200.

In another version, the system may use cross-correlation to measure the time delay of one waveform relative to one another and can generally be represented by:

$$\text{Equation 5: } R(\tau) = \int_{-\infty}^{\infty} x(t)y(t+\tau)dt$$

where x(t) and y(t) are the two waveforms as a function of time, where $\tau$ is the time delay, and where R is the cross-correlation, which is a function of the time delay $\tau$. Unlike the FFT method, cross-correlation takes place in the time domain, so no transformations are required.

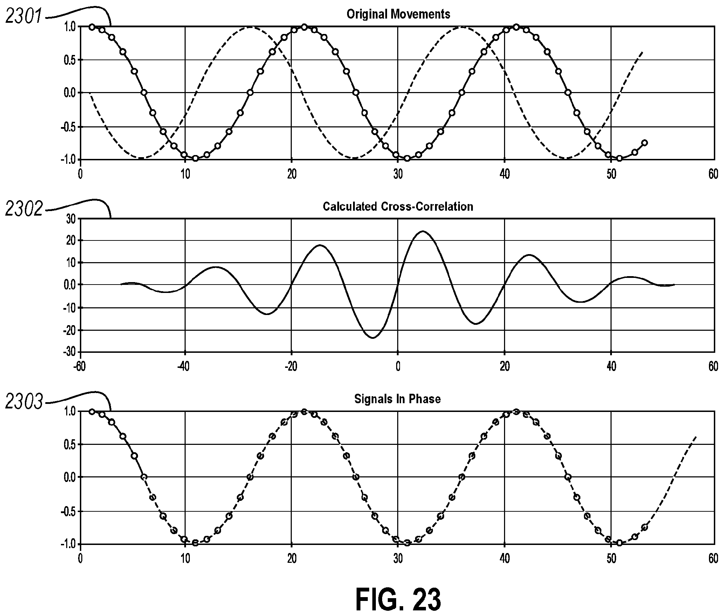

As best shown in FIG. 23, cross-correlation evaluates the time delay or shift (e.g., leading or lagging) of two waveforms, such as shown in graph 2301. As would be understood by one skilled in the art, a cross-correlation graph, such as 2302, reaches its max height, or peak, when the time delay $\tau$ is equal to zero and the waveforms are aligned on the time axis. Accordingly, based on an evaluation of the cross-correlation graph 2302 (e.g., determining the time shift from the zero axis), the system can determine if the voltage waveform is lagging or leading the current waveform. As discussed herein, specifically with reference to FIGS. 14 and 15, once the system knows whether the voltage waveform is lagging or leading the current waveform it can determine if the clamped material is capacitive in nature (e.g., tissue) or inductive in nature (e.g., non-tissue). In a further version, the system can track and evaluate the change in time delay $\tau$ over time to determine the specific type of tissue.

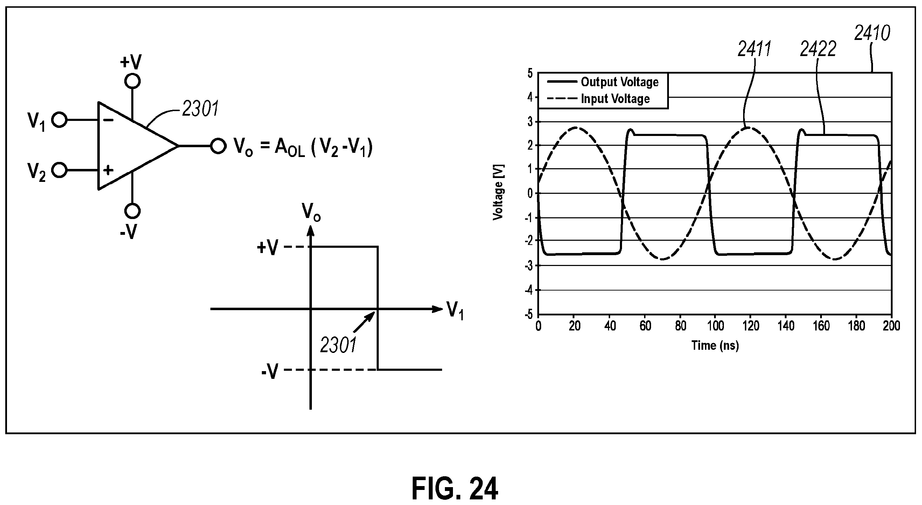

The cross-correlation method is a very robust, but somewhat time intensive method. Thus, in some versions, (e.g., where speed is valued over accuracy), the system may use the zero-crossing method. FIG. 24 shows an example waveform in graph 2411. In some versions, and as shown, the system can monitor the input voltage 2411 for any zero-crossings (i.e., the point when an alternating waveform crosses the zero value 2401, and no voltage is present). Because of the simple nature of this method, it can be carried out using minimal components (e.g., a single high-speed comparator 2402). As would be understood by one skilled in the art, a zero-crossing normally occurs twice during each cycle. Thus, by tracking the timing of the zero-crossings of two or more waveforms (e.g., the transmitted waveform 610 and the received waveform 620), the system can determine roughly how out of phase the waveforms are, and if the return signal is leading or lagging, such as shown in FIG. 15 and described above. Based on how out of phase the waveforms are (i.e., the phase angle shown at 2202), the system can determine one or more characteristics about the material clamped in the end effector 180.

According to the invention the method of analyzing the waveforms to determine a phase and/or impedance is a Pseudo Inverse Matrix Fourier (PIMF) series reconstruction. Spectral analysis using FFT may not necessarily take advantage of known information. For example, when exciting a system with a particular frequency of voltage, spectral analysis on the electrical current through the system (to thereby calculate impedance) using FFT does not capitalize on the fact that the frequency content (albeit at a different phase and magnitude) of the electrical current will be the same as the frequency content of the sent voltage signal (which is known, since it was sent). An FFT searches to estimate the phase and magnitude of the current at every single frequency in the frequency resolution of the FFT. However, in certain systems, only the frequencies that were sent in the voltage need to be analyzed.

Using a FFT, frequencies in radians per seconds (w), phases (phi), and magnitudes (A) of a signal are calculated such that the time domain signal, F(t), can be reconstructed as closely as possible using a Fourier series as follows:

$$\text{Equation 6: } f(t) = A_0 + \sum_{n=1}^{\infty}(A_n \cos w_n t + phi_n)$$

where $A_0$ is a DC offset of the signal.

Equation 6 can be expressed as follows:

$$\text{Equation 7: } f(t) = A_0 + \sum_{n=1}^{\infty}(a_n \cos w_n t + b_n \sin w_n t)$$

In this process it is assumed that the frequency content of the signal, $w_n$, is not known. However if it is assumed that $w_n$ is known (as in a system where current $w_n$ values are the same as the known inputted voltage $w_n$ values), it is possible to calculate $a_n$ and $b_n$ when $f(t)$ is known when working in the digital domain where $f(t)$ is represented by discrete points in time as $f(k_i)$ where i=0 at time zero and $i=t$ at time t where $i \in \mathbb{Z}^+$ ($i$ is an element of positive integers). The signal $f(t)$ now becomes:

$$\text{Equation 8: } f(k_i) = \begin{bmatrix} f(k_0) \\ \vdots \\ f(k_{tf}) \end{bmatrix}$$

when the rows of the column vector correspond to discrete time points of $f(t)$ @ $k_i$. Equation 7 can be used to define the following relationship which holds true for all $i \in \{0, tf\}$ and $p$ represents the discrete frequencies of the input signal:

$$\text{Equation 9: } \begin{bmatrix} f(k_0) \\ \vdots \\ f(k_{tf}) \end{bmatrix} - A_0 =$$

$$\left[ \begin{bmatrix} \cos(w_1 k_0) & \sin(w_1 k_0) \\ \vdots & \vdots \\ \cos(w_1 k_{tf}) & \sin(w_1 k_{tf}) \end{bmatrix} \quad \cdots \quad \begin{bmatrix} \cos(w_p k_0) & \sin(w_p k_0) \\ \vdots & \vdots \\ \cos(w_p k_{tf}) & \sin(w_p k_{tf}) \end{bmatrix} \right] \cdot \begin{bmatrix} \begin{bmatrix} a_1 \\ b_1 \end{bmatrix} \\ \vdots \\ \begin{bmatrix} a_p \\ b_p \end{bmatrix} \end{bmatrix}$$

The notation in Equation 9 can be reduced as follows:

$$\text{Equation 10: } \vec{f} = A \cdot \vec{x}$$

where:

$$\text{Equation 11: } \vec{f} = \begin{bmatrix} f(k_0) \\ \vdots \\ f(k_n) \end{bmatrix} - A_0$$

and:

$$\text{Equation 12: } A = \left[ \begin{bmatrix} \cos(w_1 k_0) & \sin(w_1 k_0) \\ \vdots & \vdots \\ \cos(w_1 k_n) & \sin(w_1 k_n) \end{bmatrix} \quad \cdots \quad \begin{bmatrix} \cos(w_p k_0) & \sin(w_p k_0) \\ \vdots & \vdots \\ \cos(w_p k_n) & \sin(w_p k_n) \end{bmatrix} \right]$$

are a known vector (measured) and matrix (calculated from known $w_n$) respectively. $\vec{x}$ can now be solved as follows:

$$\text{Equation 13: } \vec{x} = A^+\vec{f}$$

where $A^+$ is the pseudoinverse of $A$ which for a matrix with linearly independent columns and is:

$$\text{Equation 14: } A^+ = (A^T \cdot A)^{-1} \cdot A^T$$

Since

$$\text{Equation 15: } \vec{x} = \begin{bmatrix} \begin{bmatrix} a_1 \\ b_1 \end{bmatrix} \\ \vdots \\ \begin{bmatrix} a_p \\ b_p \end{bmatrix} \end{bmatrix}$$

is now a function of the known signal $f(k_i)$ and the known frequencies $w_n$, it can be solved for all values of $i \in \{0, tf\}$ and $n$. Once it is solved using Equation 13 for every value of $n$, the corresponding value of $A_n$ and $phi_n$ can be calculated from the trigonometric identity:

$$\text{Equation 16: } phi_n = atan2\left(\frac{b_n}{a_n}\right)$$

and

$$\text{Equation 17: } A_n = \sqrt{(a_n{}^2 + b_n{}^2)}$$

The following table represents an example of a comparison of a set of results that may be obtained using the PIMF method described above versus the FFT method:

TABLE

|  |  | Actual | PIMF | FFT |
|---|---|---|---|---|
|  |  | 15 | 13.77 | 0 |
| Phase |  | 10 | 11.59 | -3.66 |
|  |  | 18 | 18.0156 | 97.136 |
|  |  | 1 | 0.99 | 0.95 |
| Magnitude |  | 0.5 | 0.52 | 0.5 |
|  |  | 0.75 | 0.75 | 0.55 |

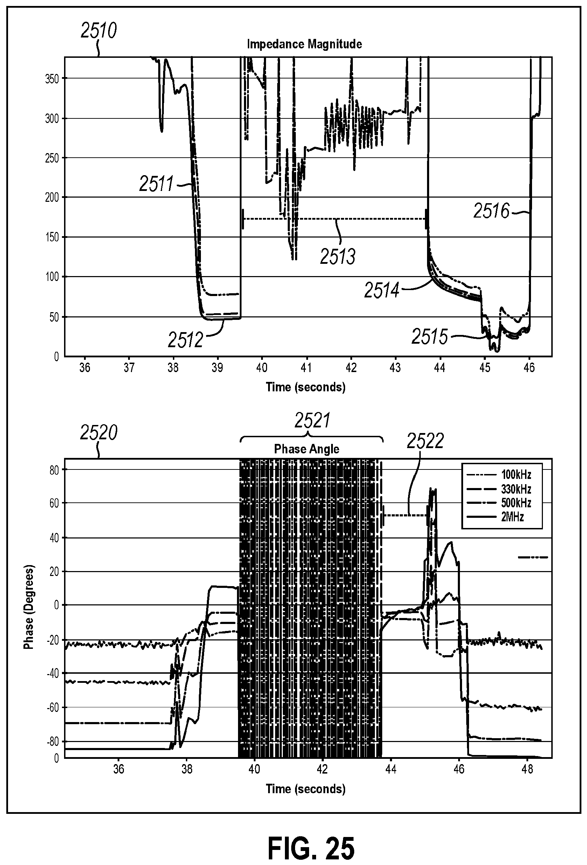

FIG. 25 shows a graphical representation of the impedance magnitude 2510 and the phase angle 2520 of a waveform that is being applied to tissue as a function of time. In some versions, and as shown, the system may make specific determinations about the surgical process based on the impedance and phase. As a non-limiting example, graph 2510 shows the impedance of the tissue which drops significantly during clamping, which is shown in period 2511. While the tissue is clamped as shown in period 2512, the impedance remains relatively stable. During this period 2512, differences in the unique signature of impedance and phase angle spectra may indicate properties of the tissue and tissue response under compression (e.g., fluid leaving tissue under strain). The therapeutic waveform is applied during the period of 2513. During this period 2513 the distal electrodes are switched to therapeutic energy delivery (e.g., to seal or cauterize the tissue), and signals may be sensed by high voltage and current therapeutic sensors. Thus, during this period 2513 of therapeutic energy delivery, the sensing signal at the distal electrodes may be inactive, which may appear as the rapid fluctuation shown in the graphical representation of the impedance magnitude 2510. The therapeutic energy delivery ultimately ceases, as shown in period 2514, with the impedance changing accordingly. The impedance can also be used to detect when the knife member 176 is fired, which is represented in period 2515.

Finally, after the procedure is complete, the end effector 180 releases the tissue, which is represented in period 2516. The phase angle graph 2520 provides a clear indication of when the therapeutic energy is being applied 2521, followed by a time delay 2522 where the tissue rests before unclamping. As discussed herein, the "rebound" time (i.e., how long certain tissues take to allow the waveform and any residual energy to dissipate from the tissue) can be used for tissue identification. Thus, by using data from one or both of the two graphs 2510, 2520, it may be possible to determine the rebound time and thus improve tissue identification.

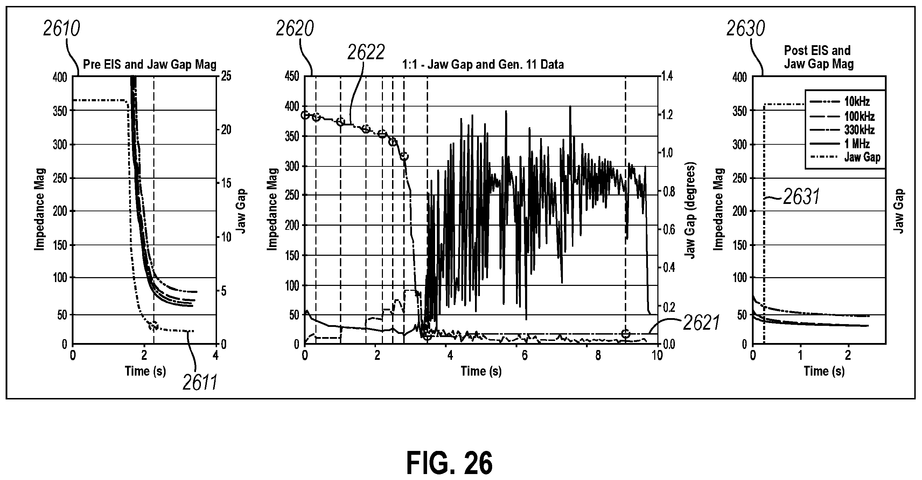

FIG. 26 shows various graphs plotting the impedance magnitude and the jaw gap vs time. The first graph 2610 shows the sensing prior to applying any therapeutic waveforms (e.g., pre-seal). Line 2611 shows the distance between the jaws 182, 184. Accordingly, as discussed herein, and shown in graph 2610 the impedance of the tissue drops as it is clamped (i.e., as the distance between the jaws is reduced). The second graph 2620 shows an example waveform during seal. Similar to graph 2610, graph 2620 also shows the jaw gap 2621. In some versions, and as shown in graph 2620, the impedance of the tissue falls, and the distance between the jaws 182, 184 decreases, during the application of therapeutic energy 2622. Finally, graph 2630 shows the recorded impedances of the sealed tissue as well as the jaw gap 2631.

VI. Miscellaneous

While the examples herein are described mainly in the context of electrosurgical instruments, it should be understood that various teachings herein may be readily applied to a variety of other types of devices. By way of example only, the various teachings herein may be readily applied to other types of electrosurgical instruments, tissue graspers, tissue retrieval pouch deploying instruments, surgical staplers, surgical clip appliers, ultrasonic surgical instruments, etc. It should also be understood that the teachings herein may be readily applied to any of the instruments described in any of the references cited herein, such that the teachings herein may be readily combined with the teachings of any of the references cited herein in numerous ways. Other types of instruments into which the teachings herein may be incorporated will be apparent to those of ordinary skill in the art.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI™ system by Intuitive Surgical, Inc., of Sunnyvale, California. Similarly, those of ordinary skill in the art will recognize that various teachings herein may be readily combined with various teachings of U.S. Pat. No. 6,783,524, entitled "Robotic Surgical Tool with Ultrasound Cauterizing and Cutting Instrument," published August 31, 2004.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

**Claims**

1. An apparatus for detecting and sealing tissue, the apparatus comprising:

   (a) a processor (602),
   (b) an end effector (180) at a distal end of a surgical instrument (100), the end effector configured to interact with a tissue of a patient, the end effector comprising:

      (i) a first jaw (182) comprising a first electrode surface (194) secured relative to the first jaw, and
      (ii) a second jaw (184) pivotably coupled with the first jaw comprising a second electrode surface (196) secured relative to the second jaw,

   wherein the first and second electrode surfaces include a plurality of electrodes;
   (c) wherein the processor is configured to:

      (i) control delivery and measurement of a non-therapeutic radio frequency (RF) signal to the plurality of electrodes, wherein the plurality of electrodes are configured to contact with the tissue of a patient;
      (ii) determine, based on the non-therapeutic RF signal, at least one characteristic of the tissue of the patient;
      (iii) determine, based on the at least one characteristic, that the plurality of electrodes are in contact with an intended tissue type;
      (iv) responsive to determining that the plurality of electrodes are in contact with the intended tissue type, control delivery of a therapeutic RF signal to the plurality of electrodes;

   **characterized in that** the processor is further configured to perform a Pseudo Inverse Matrix Fourier (PIMF) analysis on the non-therapeutic RF signal, wherein the at least one characteristic is based on the PIMF analysis.

2. The apparatus of claim 1, the first jaw further comprising a first knife pathway (192), the second jaw further comprising a second knife pathway (192), the first and second knife pathways together being configured to accommodate translation of a knife member (176) through a portion of the end effector.

3. The apparatus of claim 1 or claim 2, wherein the electrodes are in a bifurcation configuration where the electrodes are movable relative to a central axis and opposite to one another.

4. The apparatus of any one of claims 1 to 3, further comprising a switching system (601) configured to switch between the non-therapeutic RF signal and the therapeutic RF signal.

5. The apparatus of any preceding claim, further comprising:

   (a) a voltage sensor device (1210), and
   (b) a current sensor device (1220),

   wherein the processor is further configured to:

      (i) obtain, from the voltage sensor device, a send voltage, and a return voltage for the RF signal, and
      (ii) obtain, from the current sensor device, a send current and a return current for the RF signal,

   wherein the at least one characteristic is based on the send voltage, the return voltage, the send current, and the return current.

6. The apparatus of claim 5, wherein the processor is further configured to:

   (i) determine, based on the send voltage and the return voltage, a capacitive reactance of a circuit, and
   (ii) determine, based on the send voltage and the return voltage, an inductive reactance of the circuit,

wherein the at least one characteristic is based on the send voltage, the return voltage, the send current, and the return current.

7. The apparatus of claim 6, wherein the processor is further configured to:
determine, based on the capacitive reactance and the inductive reactance, an impedance of the circuit, wherein the at least one characteristic is based on the send voltage, the return voltage, the send current, and the return current.

8. The apparatus of any preceding claim, wherein the RF signal comprises a plurality of waveforms summed into a multi-waveform, wherein each of the plurality of waveforms has a unique frequency.

9. The apparatus of any preceding claim, wherein the RF signal comprises multiburst waveform with single or multiple different periods, amplitudes, or wave shapes.

10. The apparatus of any preceding claim, wherein the RF signal comprises at least one of:

(A) an amplitude modulated signal,
(B) a frequency modulated signal,
(C) a phase modulated signal,
(D) a frequency-shift keying modulation signal, or
(E) a chirp waveform.

11. The apparatus of any preceding claim, wherein the processor is further configured to perform a fast Fourier transform (FFT) on the non-therapeutic RF signal, and wherein the at least one characteristic is based on the FFT.

12. The apparatus of any preceding claim, wherein the processor is further configured to perform a cross-correlation analysis on the non-therapeutic RF signal, wherein the at least one characteristic is based on the cross-correlation analysis.

13. The apparatus of any preceding claim, wherein the processor is further configured to perform a zero-crossing analysis on the non-therapeutic RF signal, wherein the at least one characteristic is based on the zero-crossing analysis.

14. The apparatus of any preceding claim, wherein the processor is further configured to, responsive to determining that the plurality of electrodes are not in contact with the intended tissue type, perform an action selected from the group consisting of:

(i) disable delivery of a therapeutic RF signal to the plurality of electrodes,
(ii) provide a notification to a user, and
(iii) modify a surgical plan.

**Patentansprüche**

1. Vorrichtung zum Detektieren und Versiegeln von Gewebe, wobei die Vorrichtung umfasst:

(a) einen Prozessor (602),
(b) einen Endeffektor (180) an einem distalen Ende eines chirurgischen Instruments (100), wobei der Endeffektor dazu konfiguriert ist, mit einem Gewebe eines Patienten zu interagieren und wobei der Endeffektor umfasst:

(i) eine erste Backe (182), die eine erste Elektrodenfläche (194) umfasst, die relativ zur ersten Backe befestigt ist, und
(ii) eine zweite Backe (184), die schwenkbar mit der ersten Backe gekoppelt ist und eine zweite Elektrodenfläche (196) umfasst, die relativ zur zweiten Backe befestigt ist,

wobei die erste und die zweite Elektrodenfläche eine Vielzahl von Elektroden einschließen;
(c) wobei der Prozessor dazu konfiguriert ist:

(i) das Abgeben und das Messen eines nicht-therapeutischen Hochfrequenzsignals (HF-Signal) an die Vielzahl von Elektroden zu steuern, wobei die Vielzahl von Elektroden so konfiguriert ist, dass sie mit dem Gewebe eines Patienten in Kontakt kommt;

(ii) mindestens eine Eigenschaft des Gewebes des Patienten auf Basis des nicht-therapeutischen HF-Signals zu bestimmen;

(iii) basierend auf der mindestens einen Eigenschaft festzustellen, dass die Vielzahl von Elektroden mit einem beabsichtigten Gewebetyp in Kontakt steht;

(iv) als Reaktion auf die Feststellung, dass die Vielzahl von Elektroden mit dem beabsichtigten Gewebetyp in Kontakt steht, die Abgabe eines therapeutischen HF-Signals an die Vielzahl von Elektroden zu steuern;

**dadurch gekennzeichnet, dass**

der Prozessor ferner dazu konfiguriert ist, eine Pseudo-Inverse-Matrix-Fourier-Analyse (PIMF-Analyse) des nicht-therapeutischen HF-Signals durchzuführen, wobei die mindestens eine Eigenschaft auf der PIMF-Analyse beruht.

2. Vorrichtung nach Anspruch 1, wobei die erste Backe ferner einen ersten Messerweg (192) umfasst und die zweite Backe ferner einen zweiten Messerweg (192) umfasst, wobei der erste und der zweite Messerweg zusammen so konfiguriert sind, dass sie die Translation eines Messerelements (176) durch einen Abschnitt des Endeffektors ermöglichen.

3. Vorrichtung nach Anspruch 1 oder Anspruch 2, wobei die Elektroden eine Gabelungskonfiguration aufweisen, bei der die Elektroden relativ zu einer Mittelachse und einander entgegengesetzt beweglich sind.

4. Vorrichtung nach einem der Ansprüche 1 bis 3, die ferner ein Schaltsystem (601) umfasst, das zum Umschalten zwischen dem nicht-therapeutischen HF-Signal und dem therapeutischen HF-Signal konfiguriert ist.

5. Vorrichtung nach einem der vorstehenden Ansprüche, ferner umfassend:

   (a) eine Spannungssensorvorrichtung (1210) und
   (b) eine Stromsensorvorrichtung (1220),

   wobei der Prozessor ferner konfiguriert ist zum:

   (i) Empfangen einer Sendespannung und einer Rücklaufspannung für das HF-Signal von der Spannungssensorvorrichtung, und
   (ii) Empfangen eines Sendestroms und eines Rückstroms für das HF-Signal von der Stromsensorvorrichtung,

   wobei die mindestens eine Eigenschaft auf der Sendespannung, der Rücklaufspannung, dem Sendestrom und dem Rückstrom basiert.

6. Vorrichtung nach Anspruch 5, wobei der Prozessor ferner dazu konfiguriert ist:

   (i) anhand der Sendespannung und der Rücklaufspannung eine kapazitive Reaktanz eines Stromkreises zu bestimmen, und
   (ii) anhand der Sendespannung und der Rücklaufspannung eine induktive Reaktanz des Stromkreises zu bestimmen,

   wobei die mindestens eine Eigenschaft auf der Sendespannung, der Rücklaufspannung, dem Sendestrom und dem Rückstrom basiert.

7. Vorrichtung nach Anspruch 6, wobei der Prozessor ferner dazu konfiguriert ist, basierend auf der kapazitiven Reaktanz und der induktiven Reaktanz eine Impedanz des Stromkreises zu bestimmen, wobei die mindestens eine Eigenschaft auf der Sendespannung, der Rücklaufspannung, dem Sendestrom und dem Rückstrom basiert.

8. Vorrichtung nach einem der vorstehenden Ansprüche, wobei das HF-Signal eine Vielzahl von Wellenformen umfasst, die zu einer Mehrfachwellenform summiert sind, wobei jede der Vielzahl von Wellenformen eine eindeutige Frequenz aufweist.

9. Vorrichtung nach einem der vorstehenden Ansprüche, wobei das HF-Signal eine Multiburst-Wellenform mit einer oder mehreren unterschiedlichen Perioden, Amplituden oder Wellenformen umfasst.

**10.** Vorrichtung nach einem der vorstehenden Ansprüche, wobei das HF-Signal mindestens eines der Folgenden umfasst:

(A) ein amplitudenmoduliertes Signal,
(B) ein frequenzmoduliertes Signal,
(C) ein phasenmoduliertes Signal,
(D) ein Frequenzumtastungsmodulationssignal oder
(E) eine Chirp-Wellenform.

**11.** Vorrichtung nach einem der vorstehenden Ansprüche, wobei der Prozessor ferner dazu konfiguriert ist, eine schnelle Fourier-Transformation (FFT) an dem nicht-therapeutischen HF-Signal durchzuführen, und wobei die mindestens eine Eigenschaft auf der FFT basiert.

**12.** Vorrichtung nach einem der vorstehenden Ansprüche, wobei der Prozessor ferner dazu konfiguriert ist, eine Kreuzkorrelationsanalyse des nicht-therapeutischen HF-Signals durchzuführen, wobei die mindestens eine Eigenschaft auf der Kreuzkorrelationsanalyse basiert.

**13.** Vorrichtung nach einem der vorstehenden Ansprüche, wobei der Prozessor ferner dazu konfiguriert ist, eine Nulldurchgangsanalyse des nicht-therapeutischen HF-Signals durchzuführen, wobei die mindestens eine Eigenschaft auf der Nulldurchgangsanalyse basiert.

**14.** Vorrichtung nach einem der vorstehenden Ansprüche, wobei der Prozessor ferner so konfiguriert ist, dass er als Reaktion auf die Feststellung, dass die Vielzahl von Elektroden nicht mit dem beabsichtigten Gewebetyp in Kontakt steht, eine Aktion ausführt, die aus der folgenden Gruppe ausgewählt ist:

(i) Deaktivieren der Abgabe eines therapeutischen HF-Signals an die Vielzahl von Elektroden,
(ii) Benachrichtigen eines Benutzers und
(iii) Modifizieren eines Operationsplans.

**Revendications**

**1.** Appareil permettant de détecter et de sceller un tissu, l'appareil comprenant :

(a) un processeur (602),
(b) un effecteur terminal (180) au niveau d'une extrémité distale d'un instrument chirurgical (100), l'effecteur terminal étant conçu pour interagir avec un tissu d'un patient, l'effecteur terminal comprenant :

(i) une première mâchoire (182) comprenant une première surface d'électrode (194) fixée par rapport à la première mâchoire, et
(ii) une seconde mâchoire (184) accouplée de manière pivotante à la première mâchoire comprenant une seconde surface d'électrode (196) fixée par rapport à la seconde mâchoire,

dans lequel les première et seconde surfaces d'électrode comportent une pluralité d'électrodes ;
(c) dans lequel le processeur est configuré pour :

(i) commander la délivrance et la mesure d'un signal radiofréquence (RF) non thérapeutique à la pluralité d'électrodes, dans lequel la pluralité d'électrodes sont conçues pour venir en contact avec le tissu d'un patient ;
(ii) déterminer, en fonction du signal RF non thérapeutique, au moins une caractéristique du tissu du patient ;
(iii) déterminer, en fonction de l'au moins une caractéristique, que la pluralité d'électrodes sont en contact avec un type de tissu prévu ;
(iv) en réponse à la détermination que la pluralité d'électrodes sont en contact avec le type de tissu prévu, commander la délivrance d'un signal RF thérapeutique à la pluralité d'électrodes ;

**caractérisé en ce que**
le processeur est configuré en outre pour mettre en œuvre une analyse de Fourier à matrice pseudo-inverse (PIMF) sur le signal RF non thérapeutique, dans lequel l'au moins une caractéristique est en fonction de l'analyse PIMF.

**2.** Appareil selon la revendication 1, la première mâchoire comprenant en outre un premier chemin de couteau (192), la seconde mâchoire comprenant en outre un second chemin de couteau (192), les premier et second chemins de couteau étant conçus ensemble pour permettre une translation d'un élément couteau (176) à travers une partie de l'effecteur terminal.

**3.** Appareil selon la revendication 1 ou la revendication 2, dans lequel les électrodes sont dans une configuration de bifurcation où les électrodes peuvent être déplacées par rapport à un axe central et sont opposées les unes aux autres.

**4.** Appareil selon l'une quelconque des revendications 1 à 3, comprenant en outre un système de commutation (601) configuré pour commuter entre le signal RF non thérapeutique et le signal RF thérapeutique.

**5.** Appareil selon l'une quelconque revendication précédente, comprenant en outre :

(a) un dispositif capteur de tension (1210), et
(b) un dispositif capteur de courant (1220),

dans lequel le processeur est configuré en outre pour :

(i) obtenir, auprès du dispositif capteur de tension, une tension d'envoi et une tension de retour pour le signal RF, et
(ii) obtenir, auprès du dispositif capteur de courant, un courant d'envoi et un courant de retour pour le signal RF,

dans lequel l'au moins une caractéristique est en fonction de la tension d'envoi, de la tension de retour, du courant d'envoi et du courant de retour.

**6.** Appareil selon la revendication 5, dans lequel le processeur est configuré en outre pour :

(i) déterminer, en fonction de la tension d'envoi et de la tension de retour, une réactance capacitive d'un circuit, et
(ii) déterminer, en fonction de la tension d'envoi et de la tension de retour, une réactance inductive du circuit,

dans lequel l'au moins une caractéristique est en fonction de la tension d'envoi, de la tension de retour, du courant d'envoi et du courant de retour.

**7.** Appareil selon la revendication 6, dans lequel le processeur est configuré en outre pour : déterminer, en fonction de la réactance capacitive et de la réactance inductive, une impédance du circuit, dans lequel l'au moins une caractéristique est en fonction de la tension d'envoi, de la tension de retour, du courant d'envoi et du courant de retour.

**8.** Appareil selon l'une quelconque revendication précédente, dans lequel le signal RF comprend une pluralité de formes d'ondes sommées en une forme d'onde multiple, dans lequel chacune parmi la pluralité de formes d'ondes a une fréquence unique.

**9.** Appareil selon l'une quelconque revendication précédente, dans lequel le signal RF comprend forme d'onde à salves multiples avec une unique ou de multiples périodes, amplitudes ou formes d'onde différentes.

**10.** Appareil selon l'une quelconque revendication précédente, dans lequel le signal RF comprend au moins l'un parmi :

(A) un signal modulé en amplitude,
(B) un signal modulé en fréquence,
(C) un signal modulé en phase,
(D) un signal de modulation par décalage de fréquence, ou
(E) une forme d'onde chirp.

**11.** Appareil selon l'une quelconque revendication précédente, dans lequel le processeur est configuré en outre pour mettre en œuvre une transformée de Fourier rapide (FFT) sur le signal RF non thérapeutique, et dans lequel l'au moins une caractéristique est en fonction de la FFT.

**12.** Appareil selon l'une quelconque revendication précédente, dans lequel le processeur est configuré en outre pour mettre en œuvre une analyse de corrélation croisée sur le signal RF non thérapeutique, dans lequel l'au moins une caractéristique est en fonction de l'analyse de corrélation croisée.

**13.** Appareil selon l'une quelconque revendication précédente, dans lequel le processeur est configuré en outre pour mettre en œuvre une analyse de passage par le point zéro sur le signal RF non thérapeutique, dans lequel l'au moins une caractéristique est en fonction de l'analyse de passage par le point zéro.

**14.** Appareil selon l'une quelconque revendication précédente, dans lequel le processeur est configuré en outre, en réponse à la détermination que la pluralité d'électrodes ne sont pas en contact avec le type de tissu prévu, pour mettre en œuvre une action choisie dans le groupe constitué de :

(i) désactiver la délivrance d'un signal RF thérapeutique à la pluralité d'électrodes,
(ii) fournir une notification à un utilisateur, et
(iii) modifier un plan chirurgical.

**FIG. 1**

FIG. 2

**FIG. 3**

EP 4 366 638 B1

**FIG. 4**

**FIG. 5**

FIG. 6

EP 4 366 638 B1

FIG. 7

FIG. 8

**FIG. 9**

**FIG. 10**

EP 4 366 638 B1

FIG. 11

$$Gain = \left[1 + \frac{R_{Feedback,1} + R_{Feedback,2}}{R_{gain}}\right]\left[\frac{R_2}{R_1}\right]$$

**FIG. 12**

EP 4 366 638 B1

FIG. 13

**FIG. 14**

**FIG. 15**

FIG. 16

EP 4 366 638 B1

FIG. 17

FIG. 18

FIG. 19

**FIG. 20**

**FIG. 21**

2201

2202

FIG. 22

FIG. 23

FIG. 24

**FIG. 25**

FIG. 26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2335631 A2
- US 6500176 B
- US 8939974 B
- US 8888809 B
- US 9161803 B
- US 9877720 B
- US 9545253 B
- US 9526565 B
- US 8986302 B
- US 6783524 B